# EUROPEAN PATENT SPECIFICATION

(11) **EP 4 210 677 B1**
(45) Date of publication and mention of the grant of the patent: **17.06.2026**
(21) Application number: 21786682.1
(22) Date of filing: 08.09.2021
(51) Int. Cl.: A61K 9/14, A61K 9/16, A61K 31/167, A61K 47/18, A61P 31/14

(54) **NICLOSAMIDE PARTICLES AND USES THEREOF**
NICLOSAMIDPARTIKEL UND VERWENDUNGEN DAVON
PARTICULES DE NICLOSAMIDE ET LEURS UTILISATIONS

(30) Priority: 08.09.2020 US 202063075424 P
(43) Date of publication of application: 19.07.2023
(73) Proprietor: Crititech, Inc., Lawrence, KS 66044 (US)
(72) Inventor: BALTEZOR, Michael, Lawrence, KS 66044 (US); SITTENAUER, Jacob, Lawrence, KS 66044 (US); FARTHING, Joseph, Lawrence, KS 66044 (US); WILLIAMS, Mark, Lawrence, KS 66044 (US); ABARCA, Aranza, Barreda, Lawrence, KS 66044 (US)
(74) Representative: Global IP Europe Patentanwaltskanzlei
(86) International application number: PCT/US2021/049407
(87) International publication number: WO 2022/055958

(56) References cited:
- EP-A1- 3 168 211
- PADRELA LUIS ET AL: "Screening for pharmaceutical cocrystals using the supercritical fluid enhanced atomization process", THE JOURNAL OF SUPERCRITICAL FLUIDS, vol. 53, no. 1-3, 1 June 2010 (2010-06-01), AMSTERDAM, NL, pages 156 - 164, XP055869083, ISSN: 0896-8446, DOI: 10.1016/j.supflu.2010.01.010
- ANON: "TFF Pharmaceuticals Inventor Dr. Robert O. Williams III Presents Session on Repurposing Niclosamide for COVID-19 Treatment at AAPS Meeting", 3 March 2020 (2020-03-03), TFF Pharmaceuitclas, pages 1 - 2, XP055812433, Retrieved from the Internet <URL:http://pubs.acs.org/doi/pdf/10.1021/acsinfecdis.0c00052> [retrieved on 20210610], DOI: 10.1021/acsinfecdis.0c00052

## Description

### Background

Niclosamide is an FDA approved oral drug for treating helminth infections, and has been shown to inhibit multiple viruses, including but not limited to SARS- and MERS-CoV, influenza, Ebola, Lassa, Zika, and SARS-CoV-2. Niclosamide is a potent inhibitor of the TMEM16A plasma membrane ion channel, which is expressed in and affects the physiology of airway epithelial cells, the key cell type that is infected by SARS-CoV-2, leading to coronavirus disease 2019 (COVID-19). Niclosamide blocks TMEM16A-mediated cellular calcium signals to robustly bronchodilate airways, inhibit mucus secretion and reduce inflammation in the airways, representing a promising new treatment for uncontrolled asthma and COPD, among other indications. However, a significant limitation to Niclosamide therapy is that high doses of the oral formulation results in only low systemic drug concentrations making this route intractable for the treatment of respiratory infections. EP 3168211 A1 discloses compositions comprising niclosamide co-crystals and also co-crystals with urea.

### Summary

In one aspect, the disclosure provides compositions, comprising particles comprising at least 90% niclosamide by weight, or a pharmaceutically acceptable salt, hydrate, or solvate thereof, wherein the particles have one or more of:
(i) a mean bulk density (not tapped) of less than 0.40 g/cm³; and/or
(ii) a mean tapped density of less than 0.55 g/cm³.

In one embodiment, the particles have a mean bulk density (not tapped) of less than 0.20 g/cm³ and a mean tapped density of less than 0.20 g/cm³. In another embodiment, the particles have a mean bulk density (not tapped) of less than 0.15 g/cm³; and a mean tapped density of less than 0.15 g/cm³. In a further embodiment, the particles have (a) a mean bulk density (not tapped) of between about 0.02 g/cm³ and 0.30 g/cm³ and a mean tapped density of between about 0.02 g/cm³ and 0.30 g/cm³, (b) a mean bulk density (not tapped) of between about 0.03 g/cm³ and 0.30 g/cm³ and a mean tapped density of between about 0.03 g/cm³ and 0.30 g/cm³, (c) a mean bulk density (not tapped) of between about 0.04 g/cm³ and 0.30 g/cm³ and a mean tapped density of between about 0.04 g/cm³ and 0.30 g/cm³, or (d) a mean bulk density (not tapped) of between about 0.05 g/cm³ and 0.30 g/cm³ and a mean tapped density of between about 0.05 g/cm³ and 0.30 g/cm³. In one embodiment, the particles have a specific surface area (SSA) of at least about 5 m²/g. In another embodiment, the particles have a specific surface area (SSA) of between 3.6 m²/g and about 50 m²/g,

In one embodiment, the particles have:
(i) a mean bulk density (not tapped) of less than 0.40 g/cm³;
(ii) a mean tapped density of less than 0.55 g/cm³; and
(iii) a specific surface area (SSA) of at least 3.6 m²/g.

In another embodiment, the particles have:
(i) a mean bulk density (not tapped) of less than 0.40 g/cm³;
(ii) a mean tapped density of less than 0.55 g/cm³; and
(iii) a specific surface area (SSA) of at least 5 m²/g.

In a further embodiment, the particles have a mean particle size by volume distribution of between about of between about 0.1 µm and about 10.5 µm, of between about 0.1 µm and about 8 µm, between about 0.3 µm and about 7µm, between about 0.5 µm and about 6 µm.

In one embodiment, the particles comprise at least 90% niclosamide by weight. In another embodiment, the particles comprise at least 95% niclosamide by weight, or a pharmaceutically acceptable salt, hydrate, or solvate thereof. In a further embodiment, the particles comprise co-crystals of niclosamide with urea, nicotinamide, or pharmaceutically acceptable salts, hydrates, or solvate thereof, wherein the co-crystals comprise at least 95% by weight of the particles. In one embodiment, the particles comprise co-crystals of niclosamide with urea or nicotinamide, wherein the co-crystals comprise at least 95% by weight of the particles

In one embodiment, the particles comprise co-crystals of
(a) niclosamide or a pharmaceutically acceptable salt, hydrate, or solvate thereof; and
(b) urea or a pharmaceutically acceptable salts, hydrates, or solvate thereof;
wherein the co-crystals comprise at least 96%, 97%, 98%, 99%, or 100% by weight of the particles, and wherein the urea: niclosamide molar ratio present in the particles is between 3:1 and 1:3.

In another embodiment, the particles comprise co-crystals of
(a) niclosamide or a pharmaceutically acceptable salt, hydrate, or solvate thereof; and
(b) nicotinamide or a pharmaceutically acceptable salts, hydrates, or solvate thereof;
wherein the co-crystals comprise at least 96%, 97%, 98%, 99%, or 100% by weight of the particles and wherein the nicotinamide: niclosamide molar ratio present in the particles is between 3:1 and 1:3.

In one embodiment, the composition comprises a suspension further comprising a pharmaceutically acceptable aqueous carrier, and /or wherein the composition further comprises one or more components selected from the group consisting of polysorbate, methylcellulose, polyvinylpyrrolidone, mannitol, and hydroxypropyl methylcellulose. In another embodiment, the composition is formulated for pulmonary, intramuscular, subcutaneous, or intraperitoneal administration. In a further embodiment, the niclosamide particles or suspensions thereof are formulated as an dry powder inhaler aerosol, a nebulized suspension, or wherein the niclosamide particles or suspensions thereof are suspended in a suitable propellant system (including but not limited to hydrofluoroalkanes (HFAs) containing at least one liquefied gas in a pressurized container sealed with a metering valve.

In another aspect, the disclosure provides methods for treating or limiting development of a disorder including but not limited to parasitic infections, viral infections (including but not limited to including to coronaviruses (SARS, MERS, SARS-CoV-2), influenza, Ebola virus, Lassa virus, Zika virus, Dengue virus, West Nile and Japanese encephalitis virus, Chikungunya virus, Sindbis virus, Ross River virus, Semliki forest virus ), Yellow Fever virus, Rabies virus, herpes simplex virus, hepatitis C virus, rhinovirus, and coxsackivirus, bacterial infections (including but not limited to drug-resistant Mycobacterium tuberculosis, Mycobacterium abscessus, methicillin-resistant Staphylococcus aureus and biofilms, Pseudomonas aeruginosa and biofilms, vancomycin-resistant enterococci, multidrug resistant gram-negative bacteria, and anthrax , asthma, chronic obstructive pulmonary disease (COPD), cystic fibrosis, bronchiectasis, pulmonary and renal fibrosis, pulmonary hypertension, idiopathic pulmonary arterial hypertension (IPAH), acute lung injury (ALI), acute respiratory distress syndrome (ARDS), eosinophilic esophagitis, stroke, ischemic/reperfusion injury, pain, , psoriasis and atopic dermatitis, rheumatoid arthritis, graft-versus host disease and systemic sclerosis, secretory diarrhea, diabetes, kidney disease (including ADPKD), endometriosis, and tocolytic for spontaneous preterm labor, comprising administering to a subject in need thereof an amount effective to treat or limit development of the disorder of the composition or suspension of any embodiment or combination of embodiments of the disclosure. In one embodiment, the composition is administered via the pulmonary, intramuscular, or subcutaneous route. In one embodiment, the pulmonary administration comprises dry powder inhalation using a suitable dry powder inhaler device. In another embodiment, the pulmonary administration comprises nebulization, and wherein the nebulization results in pulmonary delivery to the subject of aerosol droplets of the particles or suspension thereof. In a further embodiment, the particles or suspensions thereof are aerosolized for administration, and the aerosolization results in aerosol droplets having a mass median aerodynamic diameter (MMAD) of between about 0.5 µm to about 6 µm diameter, or between about 1 µm to about 5 µm, about 1 µm to about 4 µm, about 1 µm to about 3 µm, about 2 µm to about 5 µm, about 2 µm to about 4 µm or about 2 µm to about 3 µm diameter.

In another aspect, the disclosure provides methods for making compound particles, comprising:
(a) introducing (i) a solution comprising at least one solvent selected from the group consisting of acetone, ethanol, methanol, dichloromethane, hexafluroisoproryol alcohol, trifluroethanol, dimethylsulfoxide, tetrahydrofuran (THF), dimethylformamide or combinations thereof, and at least one solute comprising niclosamide or a pharmaceutically acceptable salt, hydrate, or solvate thereof into a nozzle inlet; either alone or mixed with urea, nicotinamide, or a pharmaceutically acceptable salt, hydrate, or solvate thereof; and (ii) a compressed fluid into an inlet of a vessel defining a pressurizable chamber;
(b) passing the solution out of a nozzle orifice and into the pressurizable chamber to produce an output stream of atomized droplets, wherein the nozzle orifice is located between 2 mm and 20 mm from a sonic energy source located within the output stream, wherein the sonic energy source produces sonic energy with an amplitude between 10% and 100% during the passing, and wherein the nozzle orifice has a diameter of between 20 µm and 125 µm;
(c) contacting the atomized droplets with the compressed fluid, to cause depletion of the solvent from the atomized droplets, to produce compound particles of any embodiment or combination of embodiments disclosed herein,
wherein steps (a), (b), and (c) are carried out under supercritical temperature and pressure for the compressed fluid.

### Description of Figures

Figure 1. Scanning electron micrograph of raw material niclosamide (A) 2500X magnification, (B) 10000X magnification.
Figure 2. Scanning electron micrograph of SC13 - Low Sonication Small Batch Production - 1:1 Acetone:Ethanol - 35 mg/mL; (A) 2500X magnification, (B) 10000X magnification.
Figure 3. Scanning electron micrograph of SC20 - Co-Precipitation 1:1 Nicotinamide:Niclosamide - 4mg/mL; (A) 2500X magnification, (B) 10000X magnification
Figure 4. Scanning electron micrograph of SC27 - High Sonication 3:2 Urea:Niclosamide - 25 mg/mL; (A) 2500X magnification, (B) 10000X magnification.
Figure 5. Scanning electron micrograph of SC31 - Low Pressure Niclosamide Ethanolamine; (A) 1000X magnification, (B) 5000X magnification.
Figure 6. Scanning electron micrograph of SC58 - Niclosamide 10gm with THF; (A) 1000X magnification, (B) 5000X magnification.

### Detailed Description

. As used herein, the singular forms "a", "an" and "the" include plural referents unless the context clearly dictates otherwise. All embodiments of any aspect of the disclosure can be used in combination, unless the context clearly dictates otherwise.

As used herein, "about" means +/- 5% of the recited value.

In one aspect, the disclosure provides compositions, particles comprising at least 90% niclosamide by weight, or a pharmaceutically acceptable salt, hydrate, or solvate thereof, wherein the particles have
(i) a mean bulk density (not tapped) of less than 0.40 g/cm³; and/or
(ii) a mean tapped density of less than 0.55 g/cm³.

Niclosamide is poorly absorbed from the GI track and has substantial toxicity when administered intravenously (Andrews et al., 1982; Schweizer et al., 2018). The disclosure provides unique low bulk density, high surface area particles of niclosamide either alone or in co-crystals that provide significantly improved dissolution properties, enhanced residency times, and greater concentrations at a targeted body location, and can be administered, for example, via pulmonary, intramuscular, intraperitoneal, and subcutaneous routes as deemed appropriate for treating a given disease. Thus, the present disclosure provides niclosamide particles and suspensions thereof having significantly improved therapeutic properties to previous niclosamide therapeutics.

As used herein, "niclosamide" includes any ionization state of niclosamide, including base, acid, and neutral states. In one embodiment, the niclosamide is in the neutral state.

### Structure of niclosamide

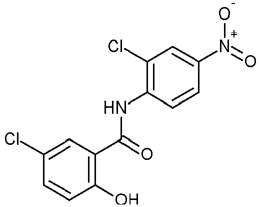

The particles comprise at least 90% niclosamide by weight, or a pharmaceutically acceptable salt, hydrate, or solvate thereof. In various embodiments, the particles comprise at least 90%, or 95% niclosamide by weight, or a pharmaceutically acceptable salt, hydrate, or solvate thereof. In other embodiments, the particles comprise co-crystals of niclosamide with urea, nicotinamide, or pharmaceutically acceptable salts, hydrates, or solvate thereof. In these embodiments, the co-crystals comprise at least 95% by weight of the particles. In various embodiments, the niclosamide, or co-crystals thereof with urea or nicotinamide comprise at least 96%, 97%, 98%, 99%, or 100% by weight of the particles.

In one embodiment, the particles comprise co-crystals of
(a) niclosamide, pharmaceutically acceptable salt, hydrate, or solvate thereof; and
(b) urea, or pharmaceutically acceptable salts, hydrates, or solvate thereof;
wherein the co-crystals comprise at least 96%, 97%, 98%, 99%, or 100% by weight of the particles.

In another embodiment, the particles comprise co-crystals of
(a) niclosamide; and
(b) urea;
wherein the co-crystals comprise at least 96%, 97%, 98%, 99%, or 100% by weight of the particles.

In these embodiments, the niclosamide and urea may be present in the particles in any molar ratio so long as the particles comprise at least 90% niclosamide by weight. In various embodiments, the urea: niclosamide molar ratio present in the particles may be between 3:1 and 1:3, or may be 1:2 or 1:3.

In another embodiment, the particles comprise co-crystals of
(a) niclosamide, pharmaceutically acceptable salt, hydrate, or solvate thereof; and
(b) nicotinamide, or pharmaceutically acceptable salts, hydrates, or solvate thereof;
wherein the co-crystals comprise at least 96%, 97%, 98%, 99%, or 100% by weight of the particles.

In a further embodiment, the particles comprise co-crystals of
(a) niclosamide; and
(b) nicotinamide;
wherein the co-crystals comprise at least 96%, 97%, 98%, 99%, or 100% by weight of the particles.

In these embodiments, the niclosamide and nicotinamide may be present in the particles in any molar ratio so long as the particles comprise at least 90% niclosamide by weight.

The inventors have unexpectedly been able to produce niclosamide particles, that have a mean bulk density (not tapped) of less than 0.40 g/cm³, a mean tapped density of less than 0.55 g/cm³, and/or a specific surface area (SSA) of at least 3.6 m²/g. The bulk densities are significantly less than previous niclosamide particles, while the SSA is significantly greater than previous niclosamide particles. The increased specific surface area and/or decreased bulk density of the niclosamide particles results in significant increases in dissolution rate compared to the micronized niclosamide products used for comparison. Dissolution takes place only at a solid/liquid interface. Therefore, increased specific surface area will increase the dissolution rate due to a larger number of molecules on the surface of the particle having contact with the dissolution media. The bulk density takes into account the macrostructure and inter-particle space of a powder. Parameters that contribute to the bulk density include particle size distribution, particle shape, and the affinity of the particles for each other (i.e., agglomeration). Lower powder bulk densities yield faster dissolution rates. This is due to the ability of the dissolution media to more readily penetrate the interstitial or inter-particle spaces and have greater contact with the surface of the particles. Therefore, each of the increased specific surface area and the decreased bulk density will result in the significant increase in dissolution rate for the niclosamide particles of the disclosure compared to micronized niclosamide product used for comparison.

As used herein, the bulk density (not tapped) of a particle is the ratio of the mass to the volume (including the interparticulate void volume) of an untapped powder sample.

As used herein, the tapped density of a particle is obtained by mechanically tapping a graduated cylinder containing the sample until little further volume change is observed.

In one embodiment, the particles of the disclosure have a mean bulk density (not tapped) of less than 0.40 g/cm³. In various embodiments the particles have a mean bulk density (not tapped) of less than 0.38 g/cm³, 0.35 g/cm³, 0.33 g/cm³, 0.30 g/cm³, 0.28 g/cm³, 0.35 g/cm³, 0.23 g/cm³, 0.20 g/cm³, 0.18 g/cm³, 0.15 g/cm³, 0.13 g/cm³, 0.10 g/cm³, 0.08 g/cm³, or 0.05 g/cm³. In various further embodiments, the particles have a mean bulk density (not tapped) of between about 0.02 g/cm³ and 0.40 g/cm³, about 0.02 g/cm³ and 0.38 g/cm³, about 0.02 g/cm³ and 0.35 g/cm³, about 0.02 g/cm³ and 0.33 g/cm³, about 0.02 g/cm³ and 0.30 g/cm³, about 0.02 g/cm³ and 0.28 g/cm³, about 0.02 g/cm³ and 0.25 g/cm³, about 0.02 g/cm³ and 0.23 g/cm³, about 0.02 g/cm³ and 0.20 g/cm³, about 0.02 g/cm³ and 0.18 g/cm³, about 0.02 g/cm³ and 0.15 g/cm³, about 0.02 g/cm³ and 0.13 g/cm³, about 0.02 g/cm³ and 0.10 g/cm³, about 0.02 g/cm³ and 0.08 g/cm³, about 0.03 g/cm³ and 0.40 g/cm³, about 0.03 g/cm³ and 0.38 g/cm³, about 0.03 g/cm³ and 0.35 g/cm³, about 0.03 g/cm³ and 0.33 g/cm³, about 0.3 g/cm³ and 0.30 g/cm³, about 0.03 g/cm³ and 0.28 g/cm³, about 0.03 g/cm³ and 0.25 g/cm³, about 0.03 g/cm³ and 0.23 g/cm³, about 0.03 g/cm³ and 0.20 g/cm³, about 0.03 g/cm³ and 0.18 g/cm³, about 0.03 g/cm³ and 0.15 g/cm³, about 0.03 g/cm³ and 0.13 g/cm³, about 0.03 g/cm³ and 0.10 g/cm³, about 0.03 g/cm³ and 0.08 g/cm³, 0.04 g/cm³ and 0.40 g/cm³, about 0.04 g/cm³ and 0.38 g/cm³, about 0.04 g/cm³ and 0.35 g/cm³, about 0.04 g/cm³ and 0.33 g/cm³, about 0.04 g/cm³ and 0.30 g/cm³, about 0.04 g/cm³ and 0.28 g/cm³, about 0.04 g/cm³ and 0.25 g/cm³, about 0.04 g/cm³ and 0.23 g/cm³, about 0.04 g/cm³ and 0.20 g/cm³, about 0.04 g/cm³ and 0.18 g/cm³, about 0.04 g/cm³ and 0.15 g/cm³, about 0.04 g/cm³ and 0.13 g/cm³, about 0.04 g/cm³ and 0.10 g/cm³, about 0.04 g/cm³ and 0.08 g/cm³, 0.05 g/cm³ and 0.40 g/cm³, about 0.05 g/cm³ and 0.38 g/cm³, about 0.05 g/cm³ and 0.35 g/cm³, about 0.05 g/cm³ and 0.33 g/cm³, about 0.05 g/cm³ and 0.30 g/cm³, about 0.05 g/cm³ and 0.28 g/cm³, about 0.05 g/cm³ and 0.25 g/cm³, about 0.05 g/cm³ and 0.23 g/cm³, about 0.05 g/cm³ and 0.20 g/cm³, about 0.05 g/cm³ and 0.18 g/cm³, about 0.05 g/cm³ and 0.15 g/cm³, about 0.05 g/cm³ and 0.13 g/cm³, or about 0.05 g/cm³ and 0.10 g/cm³.

**In** another embodiment, the particles of the disclosure have a mean tapped density of less than 0.55 g/cm³. In various embodiments the particles have a mean tapped density of less than 0.53 g/cm³, 0.50 g/cm³, 0.48 g/cm³, 0.45 g/cm³, 0.43 g/cm³, 0.40 g/cm³, 0.38 g/cm³, 0.35 g/cm³, 0.33 g/cm³, 0.30 g/cm³, 0.28 g/cm³, 0.25 g/cm³, 0.23 g/cm³, 0.20 g/cm³, 0.18 g/cm³, 0.53 g/cm³, 0.15 g/cm³, 0.13 g/cm³, 0.10 g/cm³, 0.08 g/cm³, or 0.05 g/cm³. In various further embodiments, the particles have a mean tapped density of between about 0.02 g/cm³ and 0.55 g/cm³, about 0.02 g/cm³ and 0.53 g/cm³, about 0.02 g/cm³ and 0.50 g/cm³, about 0.02 g/cm³ and 0.47 g/cm³, about 0.02 g/cm³ and 0.45 g/cm³, about 0.02 g/cm³ and 0.43 g/cm³, about 0.02 g/cm³ and 0.40 g/cm³, about 0.02 g/cm³ and 0.38 g/cm³, about 0.02 g/cm³ and 0.35 g/cm³, about 0.02 g/cm³ and 0.33 g/cm³, about 0.02 g/cm³ and 0.30 g/cm³, about 0.02 g/cm³ and 0.28 g/cm³, about 0.02 g/cm³ and 0.25 g/cm³, about 0.02 g/cm³ and 0.23 g/cm³, about 0.02 g/cm³ and 0.20 g/cm³, about 0.02 g/cm³ and 0.18 g/cm³, about 0.02 g/cm³ and 0.15 g/cm³, about 0.02 g/cm³ and 0.13 g/cm³, about 0.02 g/cm³ and 0.10 g/cm³, about 0.02 g/cm³ and 0.08 g/cm³, about 0.03 g/cm³ and 0.55 g/cm³, about 0.03 g/cm³ and 0.53 g/cm³, about 0.03 g/cm³ and 0.50 g/cm³, about 0.03 g/cm³ and 0.47 g/cm³, about 0.03 g/cm³ and 0.45 g/cm³, about 0.03 g/cm³ and 0.43 g/cm³, about 0.03 g/cm³ and 0.40 g/cm³, about 0.03 g/cm³ and 0.38 g/cm³, about 0.03 g/cm³ and 0.35 g/cm³, about 0.03 g/cm³ and 0.33 g/cm³, about 0.3 g/cm³ and 0.30 g/cm³, about 0.03 g/cm³ and 0.28 g/cm³, about 0.03 g/cm³ and 0.25 g/cm³, about 0.03 g/cm³ and 0.23 g/cm³, about 0.03 g/cm³ and 0.20 g/cm³, about 0.03 g/cm³ and 0.18 g/cm³, about 0.03 g/cm³ and 0.15 g/cm³, about 0.03 g/cm³ and 0.13 g/cm³, about 0.03 g/cm³ and 0.10 g/cm³, about 0.03 g/cm³ and 0.08 g/cm³, about 0.04 g/cm³ and 0.55 g/cm³, about 0.04 g/cm³ and 0.53 g/cm³, about 0.04 g/cm³ and 0.50 g/cm³, about 0.04 g/cm³ and 0.47 g/cm³, about 0.04 g/cm³ and 0.45 g/cm³, about 0.04 g/cm³ and 0.43 g/cm³, about 0.04 g/cm³ and 0.40 g/cm³, about 0.04 g/cm³ and 0.38 g/cm³, about 0.04 g/cm³ and 0.35 g/cm³, about 0.04 g/cm³ and 0.33 g/cm³, about 0.04 g/cm³ and 0.30 g/cm³, about 0.04 g/cm³ and 0.28 g/cm³, about 0.04 g/cm³ and 0.25 g/cm³, about 0.04 g/cm³ and 0.23 g/cm³, about 0.04 g/cm³ and 0.20 g/cm³, about 0.04 g/cm³ and 0.18 g/cm³, about 0.04 g/cm³ and 0.15 g/cm³, about 0.04 g/cm³ and 0.13 g/cm³, about 0.04 g/cm³ and 0.10 g/cm³, about 0.05 g/cm³ and 0.55 g/cm³, about 0.05 g/cm³ and 0.53 g/cm³, about 0.05 g/cm³ and 0.50 g/cm³, about 0.05 g/cm³ and 0.47 g/cm³, about 0.05 g/cm³ and 0.45 g/cm³, about 0.05 g/cm³ and 0.43 g/cm³, about 0.05 g/cm³ and 0.40 g/cm³, about 0.05 g/cm³ and 0.38 g/cm³, about 0.05 g/cm³ and 0.35 g/cm³, about 0.05 g/cm³ and 0.33 g/cm³, about 0.05 g/cm³ and 0.30 g/cm³, about 0.05 g/cm³ and 0.28 g/cm³, about 0.05 g/cm³ and 0.25 g/cm³, about 0.05 g/cm³ and 0.23 g/cm³, about 0.05 g/cm³ and 0.20 g/cm³, about 0.05 g/cm³ and 0.18 g/cm³, about 0.05 g/cm³ and 0.15 g/cm³, about 0.05 g/cm³ and 0.13 g/cm³, or about 0.05 g/cm³ and 0.10 g/cm³.

As used herein, the "specific surface area" is the total surface area of the niclosamide particle per unit of niclosamide mass as measured by the Brunauer-Emmett-Teller ("BET") isotherm (i.e.: the BET SSA). As will be understood by those of skill in the art, the SSA is determined on a per gram basis and takes into account both agglomerated and non-agglomerated niclosamide particles in the composition. The BET specific surface area test procedure is a compendial method included in both the United States Pharmacopeia and the European Pharmacopeia. In one embodiment, the niclosamide particles have a specific surface area (SSA) of at least 3.6 m²/g. In another embodiment, the niclosamide particles have an SSA of at least 5 m²/g. In various further embodiments, the niclosamide particles have a SSA of at least 4.0 m²/g, 4.5 m²/g, 5.0 m²/g. 5.5 m²/g, 6 m²/g, 6.5 m²/g, 7 m²/g, 7.5 m²/g, 8 m²/g, 8.5 m²/g, 9 m²/g, 9.5 m²/g, 10 m²/g, 10.5 m²/g, 11 m²/g, 11.5 m²/g, 12 m²/g, 12.5 m²/g, 13 m²/g, or 13.5 m²/g. In further embodiments, the niclosamide particles have a SSA of between 3.6 m²/g and about 50 m²/g, 4 m²/g and about 50 m²/g, 4.5 m²/g and about 50 m²/g, 5 m²/g and about 50 m²/g, 5 m²/g and about 45 m²/g, 5 m²/g and about 40 m²/g, 5 m²/g and about 35 m²/g, 5 m²/g and about 33 m²/g, 5 m²/g and about 30 m²/g, 5 m²/g and about 28 m²/g, 5 m²/g and about 25 m²/g, 5 m²/g and about 23 m²/g, 5 m²/g and about 20 m²/g, 5 m²/g and about 18 m²/g, 5 m²/g and about 15 m²/g, 6 m²/g and about 50 m²/g, 6 m²/g and about 45 m²/g, 6 m²/g and about 40 m²/g, 6 m²/g and about 35 m²/g, 6 m²/g and about 33 m²/g, 6 m²/g and about 30 m²/g, 6 m²/g and about 28 m²/g, 6 m²/g and about 25 m²/g, 6 m²/g and about 23 m²/g, 6 m²/g and about 20 m²/g, 6 m²/g and about 18 m²/g, 6 m²/g and about 15 m²/g, 7 m²/g and about 50 m²/g, 7 m²/g and about 45 m²/g, 7 m²/g and about 40 m²/g, 7 m²/g and about 35 m²/g, 7 m²/g and about 33 m²/g, 7 m²/g and about 30 m²/g, 7 m²/g and about 28 m²/g, 7 m²/g and about 25 m²/g, 7 m²/g and about 23 m²/g, 7 m²/g and about 20 m²/g, 7 m²/g and about 18 m²/g, 7 m²/g and about 15 m²/g, 8 m²/g and about 50 m²/g, 8 m²/g and about 45 m²/g, 8 m²/g and about 40 m²/g, 8 m²/g and about 35 m²/g, 8 m²/g and about 33 m²/g, 8 m²/g and about 30 m²/g, 8 m²/g and about 28 m²/g, 8 m²/g and about 25 m²/g, 8 m²/g and about 23 m²/g, 8 m²/g and about 20 m²/g, 8 m²/g and about 18 m²/g, 8 m²/g and about 15 m²/g, 9 m²/g and about 50 m²/g, 9 m²/g and about 45 m²/g, 9 m²/g and about 40 m²/g, 9 m²/g and about 35 m²/g, 9 m²/g and about 33 m²/g, 9 m²/g and about 30 m²/g, 9 m²/g and about 28 m²/g, 9 m²/g and about 25 m²/g, 9 m²/g and about 23 m²/g, 9 m²/g and about 20 m²/g, 9 m²/g and about 18 m²/g, 9 m²/g and about 15 m²/g, 10 m²/g and about 50 m²/g, 10 m²/g and about 45 m²/g, 10 m²/g and about 40 m²/g, 10 m²/g and about 35 m²/g, 10 m²/g and about 33 m²/g, 10 m²/g and about 30 m²/g, 10 m²/g and about 28 m²/g, 10 m²/g and about 25 m²/g, 10 m²/g and about 23 m²/g, 10 m²/g and about 20 m²/g, 10 m²/g and about 18 m²/g, or 10 m²/g and about 15 m²/g.

In one embodiment, the niclosamide particles have a mean particle size by volume distribution of from about 0.1 micron to about 10.5 microns in diameter. In another embodiment, the niclosamide particles have a mean particle size by volume distribution of from about 0.1 micron to about 8 microns in diameter. In some embodiments, the niclosamide particles have a mean particle size by volume distribution of between about 0.3 µm and about 7µm, or between about 0.5 µm and about 6 µm. The niclosamide particles are in a size range where they are less likely to be carried out of the site of administration by systemic circulation or phagocytosis and yet benefit from the high specific surface area to provide enhanced solubilization and release of the drug.

In one embodiment, the particles have:
(i) a mean bulk density (not tapped) of less than 0.40 g/cm³;
(ii) a mean tapped density of less than 0.55 g/cm³; and
(iii) a specific surface area (SSA) of at least 3.6 m²/g.

In another embodiment, the particles have:
(i) a mean bulk density (not tapped) of less than 0.40 g/cm³;
(ii) a mean tapped density of less than 0.55 g/cm³; and
(iii) a specific surface area (SSA) of at least 5 m²/g.

In various further embodiments, the particles have:
(i) a mean bulk density (not tapped) of less than 0.30 g/cm³; a mean tapped density of less than 0.30 g/cm³; and a specific surface area (SSA) of at least 3.6 m²/g, 4.0 m²/g, 4.5 m²/g, 5 m²/g, 5.5 m²/g, 6 m²/g, 6.5 m²/g, 7 m²/g, 7.5 m²/g, 8 m²/g, 8.5 m²/g, 9 m²/g, 9.5 m²/g, 10 m²/g, 10.5 m²/g, 11 m²/g, 11.5 m²/g, 12 m²/g, 12.5 m²/g, 13 m²/g, or 13.5 m²/g;
(ii) a mean bulk density (not tapped) of less than 0.10 g/cm³; a mean tapped density of less than 0.10 g/cm³; and a specific surface area (SSA) of at least 3.6 m²/g, 4.0 m²/g, 4.5 m²/g, 5 m²/g, 5.5 m²/g, 6 m²/g, 6.5 m²/g, 7 m²/g, 7.5 m²/g, 8 m²/g, 8.5 m²/g, 9 m²/g, 9.5 m²/g, 10 m²/g, 10.5 m²/g, 11 m²/g, 11.5 m²/g, 12 m²/g, 12.5 m²/g, 13 m²/g, or 13.5 m²/g;
(iii) a mean bulk density (not tapped) of less than 0.02 g/cm³; a mean tapped density of less than 0.02 g/cm³; and a specific surface area (SSA) of at least 3.6 m²/g, 4.0 m²/g, 4.5 m²/g, 5 m²/g, 5.5 m²/g, 6 m²/g, 6.5 m²/g, 7 m²/g, 7.5 m²/g, 8 m²/g, 8.5 m²/g, 9 m²/g, 9.5 m²/g, 10 m²/g, 10.5 m²/g, 11 m²/g, 11.5 m²/g, 12 m²/g, 12.5 m²/g, 13 m²/g, or 13.5 m²/g;
(iv) a mean bulk density (not tapped) of less than 0.30 g/cm³; a mean tapped density of less than 0.30 g/cm³; and a specific surface area (SSA) of between 3.6 m²/g and about 50 m²/g, 4 m²/g and about 50 m²/g, 4.5 m²/g and about 50 m²/g, 5 m²/g and about 50 m²/g, 5 m²/g and about 45 m²/g, 5 m²/g and about 40 m²/g, 5 m²/g and about 35 m²/g, 5 m²/g and about 33 m²/g, 5 m²/g and about 30 m²/g, 5 m²/g and about 28 m²/g, 5 m²/g and about 25 m²/g, 5 m²/g and about 23 m²/g, 5 m²/g and about 20 m²/g, 5 m²/g and about 18 m²/g, 5 m²/g and about 15 m²/g, 6 m²/g and about 50 m²/g, 6 m²/g and about 45 m²/g, 6 m²/g and about 40 m²/g, 6 m²/g and about 35 m²/g, 6 m²/g and about 33 m²/g, 6 m²/g and about 30 m²/g, 6 m²/g and about 28 m²/g, 6 m²/g and about 25 m²/g, 6 m²/g and about 23 m²/g, 6 m²/g and about 20 m²/g, 6 m²/g and about 18 m²/g, 6 m²/g and about 15 m²/g, 7 m²/g and about 50 m²/g, 7 m²/g and about 45 m²/g, 7 m²/g and about 40 m²/g, 7 m²/g and about 35 m²/g, 7 m²/g and about 33 m²/g, 7 m²/g and about 30 m²/g, 7 m²/g and about 28 m²/g, 7 m²/g and about 25 m²/g, 7 m²/g and about 23 m²/g, 7 m²/g and about 20 m²/g, 7 m²/g and about 18 m²/g, 7 m²/g and about 15 m²/g, 8 m²/g and about 50 m²/g, 8 m²/g and about 45 m²/g, 8 m²/g and about 40 m²/g, 8 m²/g and about 35 m²/g, 8 m²/g and about 33 m²/g, 8 m²/g and about 30 m²/g, 8 m²/g and about 28 m²/g, 8 m²/g and about 25 m²/g, 8 m²/g and about 23 m²/g, 8 m²/g and about 20 m²/g, 8 m²/g and about 18 m²/g, 8 m²/g and about 15 m²/g, 9 m²/g and about 50 m²/g, 9 m²/g and about 45 m²/g, 9 m²/g and about 40 m²/g, 9 m²/g and about 35 m²/g, 9 m²/g and about 33 m²/g, 9 m²/g and about 30 m²/g, 9 m²/g and about 28 m²/g, 9 m²/g and about 25 m²/g, 9 m²/g and about 23 m²/g, 9 m²/g and about 20 m²/g, 9 m²/g and about 18 m²/g, 9 m²/g and about 15 m²/g, 10 m²/g and about 50 m²/g, 10 m²/g and about 45 m²/g, 10 m²/g and about 40 m²/g, 10 m²/g and about 35 m²/g, 10 m²/g and about 33 m²/g, 10 m²/g and about 30 m²/g, 10 m²/g and about 28 m²/g, 10 m²/g and about 25 m²/g, 10 m²/g and about 23 m²/g, 10 m²/g and about 20 m²/g, 10 m²/g and about 18 m²/g, or 10 m²/g and about 15 m²/g.;
(v) a mean bulk density (not tapped) of less than 0.10 g/cm³; a mean tapped density of less than 0.10 g/cm³; and a specific surface area (SSA) of between 3.6 m²/g and about 50 m²/g, 4 m²/g and about 50 m²/g, 4.5 m²/g and about 50 m²/g, 5 m²/g and about 50 m²/g, 5 m²/g and about 45 m²/g, 5 m²/g and about 40 m²/g, 5 m²/g and about 35 m²/g, 5 m²/g and about 33 m²/g, 5 m²/g and about 30 m²/g, 5 m²/g and about 28 m²/g, 5 m²/g and about 25 m²/g, 5 m²/g and about 23 m²/g, 5 m²/g and about 20 m²/g, 5 m²/g and about 18 m²/g, 5 m²/g and about 15 m²/g, 6 m²/g and about 50 m²/g, 6 m²/g and about 45 m²/g, 6 m²/g and about 40 m²/g, 6 m²/g and about 35 m²/g, 6 m²/g and about 33 m²/g, 6 m²/g and about 30 m²/g, 6 m²/g and about 28 m²/g, 6 m²/g and about 25 m²/g, 6 m²/g and about 23 m²/g, 6 m²/g and about 20 m²/g, 6 m²/g and about 18 m²/g, 6 m²/g and about 15 m²/g, 7 m²/g and about 50 m²/g, 7 m²/g and about 45 m²/g, 7 m²/g and about 40 m²/g, 7 m²/g and about 35 m²/g, 7 m²/g and about 33 m²/g, 7 m²/g and about 30 m²/g, 7 m²/g and about 28 m²/g, 7 m²/g and about 25 m²/g, 7 m²/g and about 23 m²/g, 7 m²/g and about 20 m²/g, 7 m²/g and about 18 m²/g, 7 m²/g and about 15 m²/g, 8 m²/g and about 50 m²/g, 8 m²/g and about 45 m²/g, 8 m²/g and about 40 m²/g, 8 m²/g and about 35 m²/g, 8 m²/g and about 33 m²/g, 8 m²/g and about 30 m²/g, 8 m²/g and about 28 m²/g, 8 m²/g and about 25 m²/g, 8 m²/g and about 23 m²/g, 8 m²/g and about 20 m²/g, 8 m²/g and about 18 m²/g, 8 m²/g and about 15 m²/g, 9 m²/g and about 50 m²/g, 9 m²/g and about 45 m²/g, 9 m²/g and about 40 m²/g, 9 m²/g and about 35 m²/g, 9 m²/g and about 33 m²/g, 9 m²/g and about 30 m²/g, 9 m²/g and about 28 m²/g, 9 m²/g and about 25 m²/g, 9 m²/g and about 23 m²/g, 9 m²/g and about 20 m²/g, 9 m²/g and about 18 m²/g, 9 m²/g and about 15 m²/g, 10 m²/g and about 50 m²/g, 10 m²/g and about 45 m²/g, 10 m²/g and about 40 m²/g, 10 m²/g and about 35 m²/g, 10 m²/g and about 33 m²/g, 10 m²/g and about 30 m²/g, 10 m²/g and about 28 m²/g, 10 m²/g and about 25 m²/g, 10 m²/g and about 23 m²/g, 10 m²/g and about 20 m²/g, 10 m²/g and about 18 m²/g, or 10 m²/g and about 15 m²/g.; or
(vi) a mean bulk density (not tapped) of less than 0.05 g/cm³; a mean tapped density of less than 0.05 g/cm³; and a specific surface area (SSA) of between 3.6 m²/g and about 50 m²/g, 4 m²/g and about 50 m²/g, 4.5 m²/g and about 50 m²/g, 5 m²/g and about 50 m²/g, 5 m²/g and about 45 m²/g, 5 m²/g and about 40 m²/g, 5 m²/g and about 35 m²/g, 5 m²/g and about 33 m²/g, 5 m²/g and about 30 m²/g, 5 m²/g and about 28 m²/g, 5 m²/g and about 25 m²/g, 5 m²/g and about 23 m²/g, 5 m²/g and about 20 m²/g, 5 m²/g and about 18 m²/g, 5 m²/g and about 15 m²/g, 6 m²/g and about 50 m²/g, 6 m²/g and about 45 m²/g, 6 m²/g and about 40 m²/g, 6 m²/g and about 35 m²/g, 6 m²/g and about 33 m²/g, 6 m²/g and about 30 m²/g, 6 m²/g and about 28 m²/g, 6 m²/g and about 25 m²/g, 6 m²/g and about 23 m²/g, 6 m²/g and about 20 m²/g, 6 m²/g and about 18 m²/g, 6 m²/g and about 15 m²/g, 7 m²/g and about 50 m²/g, 7 m²/g and about 45 m²/g, 7 m²/g and about 40 m²/g, 7 m²/g and about 35 m²/g, 7 m²/g and about 33 m²/g, 7 m²/g and about 30 m²/g, 7 m²/g and about 28 m²/g, 7 m²/g and about 25 m²/g, 7 m²/g and about 23 m²/g, 7 m²/g and about 20 m²/g, 7 m²/g and about 18 m²/g, 7 m²/g and about 15 m²/g, 8 m²/g and about 50 m²/g, 8 m²/g and about 45 m²/g, 8 m²/g and about 40 m²/g, 8 m²/g and about 35 m²/g, 8 m²/g and about 33 m²/g, 8 m²/g and about 30 m²/g, 8 m²/g and about 28 m²/g, 8 m²/g and about 25 m²/g, 8 m²/g and about 23 m²/g, 8 m²/g and about 20 m²/g, 8 m²/g and about 18 m²/g, 8 m²/g and about 15 m²/g, 9 m²/g and about 50 m²/g, 9 m²/g and about 45 m²/g, 9 m²/g and about 40 m²/g, 9 m²/g and about 35 m²/g, 9 m²/g and about 33 m²/g, 9 m²/g and about 30 m²/g, 9 m²/g and about 28 m²/g, 9 m²/g and about 25 m²/g, 9 m²/g and about 23 m²/g, 9 m²/g and about 20 m²/g, 9 m²/g and about 18 m²/g, 9 m²/g and about 15 m²/g, 10 m²/g and about 50 m²/g, 10 m²/g and about 45 m²/g, 10 m²/g and about 40 m²/g, 10 m²/g and about 35 m²/g, 10 m²/g and about 33 m²/g, 10 m²/g and about 30 m²/g, 10 m²/g and about 28 m²/g, 10 m²/g and about 25 m²/g, 10 m²/g and about 23 m²/g, 10 m²/g and about 20 m²/g, 10 m²/g and about 18 m²/g, or 10 m²/g and about 15 m²/g..

In any of these various embodiments, the niclosamide particles may include, for example, at least 5 X 10⁻¹⁵ gram niclosamide or a pharmaceutically acceptable salt, hydrate, solvate, or co-crystal thereof per niclosamide particle, or between about 1 X 10⁻⁸ and about 5 X 10⁻¹⁵ gram niclosamide, or a pharmaceutically acceptable salt, hydrate, or solvate thereof per niclosamide particle.

In one embodiment, the particles are uncoated. In a further embodiment, the composition comprises a suspension further comprising a pharmaceutically acceptable aqueous carrier. The suspension of the disclosure comprises niclosamide particles and a liquid carrier. The liquid carrier can be aqueous. Even though the niclosamide particles do not include an added excipient, the liquid carrier of the suspension can comprise water and optionally one or more excipients selected from the group consisting of buffer, tonicity adjusting agent, preservative, demulcent, viscosity modifier, osmotic agent, surfactant, antioxidant, alkalinizing agent, acidifying agent, antifoaming agent, and colorant. For example, the suspension can comprise niclosamide particles, water, buffer and salt. It optionally further comprises a surfactant. In some embodiments, the suspension consists essentially of or consists of water, niclosamide particles suspended in the water and buffer. The suspension can further contain an osmotic salt.

In one embodiment, the composition further comprises one or more components selected from the group consisting of polysorbate, methylcellulose, polyvinylpyrrolidone, mannitol, and hydroxypropyl methylcellulose.

The suspension can comprise one or more surfactants. Suitable surfactants include by way of example and without limitation polysorbates, lauryl sulfates, acetylated monoglycerides, diacetylated monoglycerides, and poloxamers.

The suspension can comprise one or more tonicity adjusting agents. Suitable tonicity adjusting agents include by way of example and without limitation, one or more inorganic salts, electrolytes, sodium chloride, potassium chloride, sodium phosphate, potassium phosphate, sodium, potassium sulfates, sodium and potassium bicarbonates and alkaline earth metal salts, such as alkaline earth metal inorganic salts, e.g., calcium salts, and magnesium salts, mannitol, dextrose, glycerin, propylene glycol, and mixtures thereof.

The suspension can comprise one or more buffering agents. Suitable buffering agents include by way of example and without limitation, dibasic sodium phosphate, monobasic sodium phosphate, citric acid, sodium citrate hydrochloric acid, sodium hydroxide, tris(hydroxymethyl)aminomethane, bis(2-hydroxyethyl)iminotris-(hydroxymethyl)methane, and sodium hydrogen carbonate and others known to those of ordinary skill in the art. Buffers are commonly used to adjust the pH to a desirable range for intraperitoneal use. Usually a pH of around 5 to 9, 5 to 8, 6 to 7.4, 6.5 to 7.5, or 6.9 to 7.4 is desired.

The suspension can comprise one or more demulcents. A demulcent is an agent that forms a soothing film over a mucous membrane, such as the membranes lining the peritoneum and organs therein. A demulcent may relieve minor pain and inflammation and is sometimes referred to as a mucoprotective agent. Suitable demulcents include cellulose derivatives ranging from about 0.2 to about 2.5 % such as carboxymethylcellulose sodium, hydroxyethyl cellulose, hydroxypropyl methylcellulose, and methylcellulose; gelatin at about 0.01%; polyols in about 0.05 to about 1%, also including about 0.05 to about 1%, such as glycerin, polyethylene glycol 300, polyethylene glycol 400, polysorbate 80, and propylene glycol; polyvinyl alcohol from about 0.1 to about 4 %; povidone from about 0.1 to about 2%; and dextran 70 from about 0.1% when used with another polymeric demulcent described herein.

The suspension can comprise one or more alkalinizing agents to adjust the pH. As used herein, the term "alkalizing agent" is intended to mean a compound used to provide an alkaline medium. Such compounds include, by way of example and without limitation, ammonia solution, ammonium carbonate, potassium hydroxide, sodium carbonate, sodium bicarbonate, and sodium hydroxide and others known to those of ordinary skill in the art

The suspension can comprise one or more acidifying agents to adjust the pH. As used herein, the term "acidifying agent" is intended to mean a compound used to provide an acidic medium. Such compounds include, by way of example and without limitation, acetic acid, amino acid, citric acid, nitric acid, fumaric acid and other alpha hydroxy acids, hydrochloric acid, ascorbic acid, and nitric acid and others known to those of ordinary skill in the art.

The suspension can comprise one or more antifoaming agents. As used herein, the term "antifoaming agent" is intended to mean a compound or compounds that prevents or reduces the amount of foaming that forms on the surface of the fill composition. Suitable antifoaming agents include by way of example and without limitation, dimethicone, SIMETHICONE^{®}, octoxynol and others known to those of ordinary skill in the art.

The suspension can comprise one or more viscosity modifiers that increase or decrease the viscosity of the suspension. Suitable viscosity modifiers include methylcellulose, hydroxypropyl methycellulose, mannitol and polyvinylpyrrolidone.

The suspension can comprise one or more osmotic agents such as those used for peritoneal dialysis. Suitable osmotic agents include icodextrin (a glucose polymer), sodium chloride, potassium chloride, and salts that are also used as buffering agents.

As used herein, "pharmaceutically acceptable salts, solvates, or hydrates" of niclosamide are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the niclosamide. The term "salts" refers to the relatively non-toxic, inorganic and organic acid addition salts of niclosamide. Representative salts include the hydrobromide, hydrochloride, sulfate, bisulfate, nitrate, acetate, oxalate, valerate, oleate, palmitate, stearate, laurate, malate, borate, benzoate, lactate, phosphate, tosylate, citrate, maleate, fumarate, succinate, tartrate, naphthylate, mesylate, glucoheptonate, lactobionate, and laurylsulphonate salts, and the like. These may include cations based on the alkali and alkaline earth metals, such as sodium, lithium, potassium, calcium, magnesium, and the like, as well as non-toxic ammonium, quaternary ammonium, and amine cations including, but not limited to ammonium, tetramethylammonium, tetraethylammonium, methylamine, dimethylamine, trimethylamine, triethylamine, ethylamine, and the like. (See, for example, Berge S.M. et al., "Pharmaceutical Salts," J. Pharm. Sci., 1977;66:1-19.) In one embodiment, the pharmaceutically acceptable salt of niclosamide comprises niclosamide malate.

In one embodiment, the composition comprises a dosage form of niclosamide in suspension (i.e.: with a pharmaceutically acceptable carrier and any other components), in a dosage deemed suitable by an attending physician for an intended use. Any suitable dosage form may be used; in various non-limiting embodiments, the dosage form is adequate to provide about 0.01 mg/kg to about 50 mg/kg of body weight per day. In various further embodiments, the dosage form is adequate to provide about 0.01 mg/kg to about 45 mg/kg, about 0.01 mg/kg to about 40 mg/kg, about 0.01 mg/kg to about 35 mg/kg, about 0.01 mg/kg to about 30 mg/kg, about 0.01 mg/kg to about 25 mg/kg, about 0.01 mg/kg to about 20 mg/kg, about 0.01 mg/kg to about 15 mg/kg, about 0.01 mg/kg to about 10 mg/kg, about 0.01 mg/kg to about 5 mg/kg, or about 0.01 mg/kg to about 1 mg/kg of body weight per day. The suspension can be administered as is or can be diluted with a diluent, e.g. with saline water for injection optionally including a buffering agent and one or more other excipients, prior to administration. For example, the volume ratio of suspension to diluent might be in the range of 1:1 - 1: 100 (v/v) or other suitable ratio.

In another embodiment, the niclosamide particles or suspensions thereof for use in the treatments mentioned below are formulated for administration via the pulmonary, intramuscular, subcutaneous, or intraperitoneal route. In one embodiment where the niclosamide particles or suspensions thereof are formulated for administration via the pulmonary route, the niclosamide particles may be formulated as a dry powder. In another embodiment where the niclosamide particles or suspensions thereof are formulated for administration via the pulmonary route, the niclosamide particles may be formulated as an aerosol (i.e.: liquid droplets of a stable dispersion or suspension of the niclosamide particles in a gaseous medium). Niclosamide particles delivered by aerosol may be deposited in the airways by gravitational sedimentation, inertial impaction, and/or diffusion. Any suitable device for generating the aerosol may be used, including but not limited to pressured meter inhalers (pMDI), nebulizers, dry powder inhalers (DPI), and soft-mist inhalers.

In one embodiment where the niclosamide particles or suspensions thereof for use in the treatments mentioned below are formulated for administration via the pulmonary route, the niclosamide particles or suspensions thereof may be suspended in a suitable propellant system (including but not limited to hydrofluoroalkanes (HFAs) containing at least one liquefied gas in a pressurized container sealed with a metering valve. Actuation of the valve results in delivery of a metered dose of an aerosol spray of the niclosamide particles or suspensions thereof.

In another aspect, the disclosure provides an inventive composition as described herein, for use in the treatment of or for use in limiting development of a disorder including but not limited to parasitic infections, viral infections (including but not limited to including to coronaviruses (SARS, MERS, SARS-CoV-2), influenza, Ebola virus, Lassa virus, Zika virus, Dengue virus ), West Nile and Japanese encephalitis virus, Chikungunya virus, Sindbis virus, Ross River virus, Semliki forest virus ), Yellow Fever virus, Rabies virus, herpes simplex virus, hepatitis C virus, rhinovirus, and coxsackivirus, bacterial infections (including but not limited to drug-resistant Mycobacterium tuberculosis, Mycobacterium abscessus, methicillin-resistant Staphylococcus aureus and biofilms, Pseudomonas aeruginosa and biofilms, vancomycin-resistant enterococci, multidrug resistant gram-negative bacteria, and anthrax , asthma, chronic obstructive pulmonary disease (COPD), cystic fibrosis, bronchiectasis, pulmonary and renal fibrosis, pulmonary hypertension, idiopathic pulmonary arterial hypertension (IPAH), acute lung injury (ALI), acute respiratory distress syndrome (ARDS) , eosinophilic esophagitis, stroke, ischemic/reperfusion injury, pain, , psoriasis and atopic dermatitis, rheumatoid arthritis, graft-versus host disease and systemic sclerosis, secretory diarrhea, diabetes, kidney disease (including ADPKD), endometriosis, and tocolytic for spontaneous preterm labor.

Niclosamide has general anti-inflammatory activity, and is thus useful in treating a wide variety of inflammatory disorders by inhibiting several key pathways leading to inflammation, including Akt, MEK/ERK, mTORC1, STAT3, NF-κB, Wnt/β-catenin, and Notch signaling pathways, as discussed in the scientific literature (see the attached list of references). Niclosamide has also been identified as a potent inhibitor of the calcium-activated chloride channel, TMEM16A (Anoctamin-1, Ano1), which is expressed in, and affects the physiology of epithelial cells, smooth muscle cells and sensory neurons (Miner et al., 2019). Niclosamide's activity in inhibiting this target may explain its efficacy in treating many of the disorders listed above.

However, niclosamide is poorly absorbed from the GI track and has substantial toxicity when administered intravenously (Andrews et al., 1982; Schweizer et al., 2018). The disclosure provides unique low bulk density, high surface area particles of niclosamide either alone or in co-crystals that provide significantly improved dissolution properties combined with significantly enhanced residency times and far greater concentrations at a targeted body location, and can be used in administration , for example, via pulmonary, intramuscular, subcutaneous, and intraperitoneal routes as deemed appropriate for treating a given disease. Thus, the present disclosure provides niclosamide particles and suspensions thereof having significantly improved therapeutic properties to previous niclosamide therapeutics. Furthermore, in some embodiments the methods of the present disclosure can reduce the dosing frequency and side effects of niclosamide.

The particles for use in the above treatments may be administered via any suitable administrative route, including but not limited to the pulmonary, intramuscular, subcutaneous, or intraperitoneal route, as deemed most appropriate by attending medical personnel in light of all factors for a given subject.

The subject may be any suitable subject with a tumor, including but not limited to humans, primates, dogs, cats, horses, cattle, etc. In one embodiment, the subject is a human subject.

As used herein, "treat" or "treating" means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "limiting development" or "limiting" a disorder means prophylactic use to prevent or reduce the occurrence of one getting a disorder, such as prophylactic treatment (such as a vaccination) to avoid becoming infected with a virus.

Amounts effective for these uses depend on factors including, but not limited to, the nature of the composition (amount of niclosamide in the particles, etc.), the route of administration, the stage and severity of the disorder, the weight and general state of health of the subject, and the judgment of the prescribing physician. It will be understood that the amount of the composition of suspension of the disclosure actually administered will be determined by a physician, in the light of the above relevant circumstances. In one non-limiting embodiment, an amount effective is an amount that provides between 0.01 mg/kg to about 50 mg/kg of body weight per day.

In one embodiment, the disorder comprises a viral infection, and wherein the viral infection comprises a beta-coronavirus infection selected from the group consisting of infection by Human coronavirus HKU1, SARS-CoV (including but not limited to SARS-CoV-2), and MERS-CoV. In a specific embodiment, the beta-coronavirus infection comprises a SARS-CoV-2 infection. In one embodiment, according to the disclosure but not according to the invention, the methods treat the viral infection. In another embodiment, the methods limit development of the viral infection. In other specific embodiments, the disorder comprises asthma, COPD, and/or cystic fibrosis.

In one specific embodiment, the composition for use in the treatments above is administered via the pulmonary route. In one specific embodiment of the invention, pulmonary administration comprises inhalation of a single dose of the niclosamide particles, such as by nasal, oral inhalation, or both. The niclosamide particles can be administered in two or more separate administrations (doses). In this embodiment, the niclosamide particles may be formulated as a dry powder (either alone, as a mixture, or in a dry blend with, for example, lactose) from a dry powder inhaler or as an aerosol spray (i.e.: liquid droplets of a stable dispersion or suspension of the niclosamide particles in a gaseous medium). Niclosamide particles delivered by aerosol may be deposited in the airways by gravitational sedimentation, inertial impaction, and/or diffusion. Any suitable device for generating the aerosol may be used, including but not limited to pressured meter inhalers (pMDI), nebulizers, dry powder inhalers (DPI), and soft-mist inhalers.

In one specific embodiment, according to the disclosure but not according to the invention, the methods comprise inhalation of niclosamide particles aerosolized via nebulization. Nebulizers generally use compressed air or ultrasonic power to create inhalable aerosol droplets of the niclosamide particles or suspensions thereof. In this embodiment, according to the disclosure but not according to the invention, the nebulizing results in pulmonary delivery to the subject of aerosol droplets of the niclosamide particles or suspension thereof.

In another embodiment, according to the disclosure but not according to the invention, the methods comprise inhalation of niclosamide particles aerosolized via a pMDI, wherein the niclosamide particles or suspensions thereof are suspended in a suitable propellant system (including but not limited to hydrofluoroalkanes (HFAs) containing at least one liquefied gas in a pressurized container sealed with a metering valve. Actuation of the valve results in delivery of a metered dose of an aerosol spray of the niclosamide particles or suspensions thereof.

In such embodiments, the particles or suspensions thereof are aerosolized for administration, and the aerosolization results in aerosol droplets having a mass median aerodynamic diameter (MMAD) of between about 0.5 µm to about 6 µm diameter, or between about 1 µm to about 5 µm, about 1 µm to about 4 µm, about 1 µm to about 3 µm, about 2 µm to about 5 µm, about 2 µm to about 4 µm or about 2 µm to about 3 µm diameter.

In all embodiments, the compositions for use in the above treatments may be administered once, or multiple times as deemed appropriate by attending medical personnel in light of all factors, particularly the disorder being treated.

In another aspect, the disclosure provides methods for making compound particles, comprising:
(a) introducing (i) a solution comprising at least one solvent selected from the group consisting of acetone, ethanol, methanol, dichloromethane, hexafluroisoproryol alcohol, trifluroethanol, dimethylsulfoxide, tetrahydrofuran (THF), dimethylformamide or combinations thereof, and at least one solute comprising niclosamide or a pharmaceutically acceptable salt, hydrate, or solvate thereof into a nozzle inlet; either alone or mixed with urea, nicotinamide, or a pharmaceutically acceptable salt, hydrate, or solvate thereof; and (ii) a compressed fluid into an inlet of a vessel defining a pressurizable chamber;
(b) passing the solution out of a nozzle orifice and into the pressurizable chamber to produce an output stream of atomized droplets, wherein the nozzle orifice is located between 2 mm and 20 mm from a sonic energy source located within the output stream, wherein the sonic energy source produces sonic energy with an amplitude between 10% and 100% during the passing, and wherein the nozzle orifice has a diameter of between 20 µm and 125 µm;
(c) contacting the atomized droplets with the compressed fluid, to cause depletion of the solvent from the atomized droplets, to produce niclosamide compound particles as per any embodiment or combination of embodiments disclosed herein,
wherein steps (a), (b), and (c) are carried out under supercritical temperature and pressure for the compressed fluid.

The methods utilize a sonic energy source located directly in the output stream of the solute dissolved in the solvent. Any suitable source of sonic energy may be used that is compatible with the methods of the disclosure, including but not limited to sonic horn, a sonic probe, or a sonic plate. In various embodiments, the nozzle orifice is located between about 2 mm and about 20 mm, about 2 mm and about 18 mm, about 2 mm and about 16 mm, about 2 mm and about 14 mm, about 2 mm and about 12 mm, about 2 mm and about 10 mm, about 2 mm and about 8 mm, about 2 mm and about 6 mm, about 2 mm and about 4 mm, about 4 mm and about 20 mm, about 4 mm and about 18 mm, about 4 mm and about 16 mm, about 4 mm and about 14 mm, about 4 mm and about 12 mm, about 4 mm and about 10 mm, about 4 mm and about 8 mm, about 4 mm and about 6 mm, about 6 mm and about 20 mm, about 6 mm and about 18 mm, about 6 mm and about 16 mm, about 6 mm and about 14 mm, about 6 mm and about 12 mm, about 6 mm and about 10 mm, about 6 mm and about 8 mm, about 8 mm and about 20 mm, about 8 mm and about 18 mm, about 8 mm and about 16 mm, about 8 mm and about 14 mm, about 8 mm and about 12 mm, about 8 mm and about 10 mm, about 10 mm and about 20 mm, about 10 mm and about 18 mm, about 10 mm and about 16 mm, about 10 mm and about 14 mm, about 10 mm and about 12 mm, about 12 mm and about 20 mm, about 12 mm and about 18 mm, about 12 mm and about 16 mm, about 12 mm and about 14 mm, about 14 mm and about 20 mm, about 14 mm and about 18 mm, about 14 mm and about 16 mm, about 16 mm and about 20 mm, about 16 mm and about 18 mm, and about 18 mm and about 20 mm, from the sonic energy source. In further embodiments, the nozzle assembly of any embodiment of WO2016/197091 may be used.

Any suitable source of sonic energy may be used that is compatible with the methods of the disclosure, including but not limited to sonic horn, a sonic probe, or a sonic plate. In various further embodiments, the sonic energy source produces sonic energy with an amplitude between about 10% and about 100% of the total power that can be generated using the sonic energy source. In light of the teachings herein, one of skill in the art can determine an appropriate sonic energy source having a specific total power output to be used. In one embodiment, the sonic energy source has a total power output of between about 500 and about 900 watts; in various further embodiments, between about 600 and about 800 watts, about 650-750 watts, or about 700 watts.

In various further embodiments , the sonic energy source produces sonic energy with a power output between about 20% and about 100%, about 30% and about 100%, about 40% and about 100%, about 50% and about 100%, about 60% and about 100%, about 70% and about 100%, about 80% and about 100%, about 90% and about 100%, about 10% and about 90%, about 20% and about 90%, about 30% and about 90%, about 40% and about 90%, about 50% and about 90%, about 60% and about 90%, about 70% and about 90%, about 80% and about 90%, about 10% and about 80%, about 20% and about 80%, about 30% and about 80%, about 40% and about 80%, about 50% and about 80%, about 60% and about 80%, about 70% and about 80%, about 10% and about 70%, about 20% and about 70%, about 30% and about 70%, about 40% and about 70%, about 50% and about 70%, about 60% and about 70%, about 10% and about 60%, about 20% and about 60%, about 30% and about 60%, about 40% and about 60%, about 50% and about 60%, about 10% and about 50%, about 20% and about 50%, about 30% and about 50%, about 40% and about 50%, about 10% and about 40%, about 20% and about 40%, about 30% and about 40%, about 10% and about 30%, about 20% and about 30%, about 10% and about 20%, or about 10%, 20%, 30%, 40%, 50%, 60%, 70%, 80%, 90%, or about 100% of the total power that can be generated using the sonic energy source. In light of the teachings herein, one of skill in the art can determine an appropriate frequency to be utilized on the sonic energy source. In one embodiment, a frequency of between about 18 and about 22 kHz on the sonic energy source is utilized. In various other embodiments, a frequency of between about 19 and about 21 kHz, about 19.5 and about 20.5, or a frequency of about 20 kHz on the sonic energy source is utilized.

In various further embodiments, the nozzle orifice has a diameter of between about 20 µm and about 125 µm, about 20 µm and about 115 µm, about 20 µm and about 100 µm, about 20 µm and about 90 µm, about 20 µm and about 80 µm, about 20 µm and about 70 µm, about 20 µm and about 60 µm, about 20 µm and about 50 µm, about 20 µm and about 40 µm, about 20 µm and about 30 µm, between about 30 µm and about 125 µm, about 30 µm and about 115 µm, about 30 µm and about 100 µm, about 30 µm and about 90 µm, about 30 µm and about 80 µm, about 30 µm and about 70 µm, about 30 µm and about 60 µm, about 30 µm and about 50 µm, about 30 µm and about 40 µm, between about 40 µm and about 125 µm, about 40 µm and about 115 µm, about 40 µm and about 100 µm, about 40 µm and about 90 µm, about 40 µm and about 80 µm, about 40 µm and about 70 µm, about 40 µm and about 60 µm, about 40 µm and about 50 µm, between about 50 µm and about 125 µm, about 50 µm and about 115 µm, about 50 µm and about 100 µm, about 50 µm and about 90 µm, about 50 µm and about 80 µm, about 50 µm and about 70 µm, about 50 µm and about 60 µm, between about 60 µm and about 125 µm, about 60 µm and about 115 µm, about 60 µm and about 100 µm, about 60 µm and about 90 µm, about 60 µm and about 80 µm, about 60 µm and about 70 µm, between about 70 µm and about 125 µm, about 70 µm and about 115 µm, about 70 µm and about 100 µm, about 70 µm and about 90 µm, about 70 µm and about 80 µm, between about 80 µm and about 125 µm, about 80 µm and about 115 µm, about 80 µm and about 100 µm, about 80 µm and about 90 µm, between about 90 µm and about 125 µm, about 90 µm and about 115 µm, about 90 µm and about 100 µm, between about 100 µm and about 125 µm, about 100 µm and about 115 µm, between about 115 µm and about 125 µm, about 20 µm, 30 µm, 40 µm, 50 µm, 60 µm, 70 µm, 80 µm, 90 µm, 100 µm, 115 µm, or about 120 µm. The nozzle is inert to both the solvent and the compressed fluid used in the methods.

The solvent comprise acetone, ethanol, methanol, dichloromethane, hexafluroisoproryol alcohol, trifluroethanol, dimethylsulfoxide, dimethylformamide or combinations thereof. The solvent should comprise at least about 80%, 85%, or 90% by weight of the overall solution. In one embodiment, the solvent comprises acetone, ethanol, methanol, or combinations thereof.

The compressed fluid is capable of forming a supercritical fluid under the conditions used, and the solute that forms the particles is poorly soluble or insoluble in the compressed fluid. As is known to those of skill in the art, a supercritical fluid is any substance at a temperature and pressure above its critical point, where distinct liquid and gas phases do not exist. Steps (a), (b), and (c) of the methods of the disclosure are carried out under supercritical temperature and pressure for the compressed fluid, such that the compressed fluid is present as a supercritical fluid during these processing steps.

The compressed fluid can serve as a solvent for and can be used to remove unwanted components in the particles. Any suitable compressed fluid may be used in the methods of the disclosure; exemplary such compressed fluids are disclosed in U.S. Patent Nos. 5833891 and 5874029. In one non-limiting embodiment, suitable supercritical fluid-forming compressed fluids and/or anti-solvents can comprise carbon dioxide, ethane, propane, butane, isobutane, nitrous oxide, xenon, sulfur hexafluoride and trifluoromethane. The anti-solvent recited in step (d) to cause further solvent depletion, is a compressed fluid as defined above, and may be the same compressed fluid used in steps (a-c), or may be different. In one embodiment, the anti-solvent used in step (d) is the same as the compressed fluid used in steps (a-c). In a preferred embodiment, the compressed fluid and the anti-solvent are both super-critical carbon dioxide.

In all cases, the compressed fluid and anti-solvent should be substantially miscible with the solvent while the compound to be precipitated should be substantially insoluble in the compressed fluid, i.e., the compound, at the selected solvent/compressed fluid contacting conditions, should be no more than about 5% by weight soluble in the compressed fluid or anti-solvent, and preferably is essentially completely insoluble.

The supercritical conditions used in the methods of the disclosure are typically in the range of from 1X to about 1.4X, or 1X to about 1.2X of the critical temperature of the supercritical fluid, and from 1X to about 7X, or 1X to about 2X, of the of the supercritical pressure for the compressed fluid.

It is well within the level of those of skill in the art to determine the critical temperature and pressure for a given compressed fluid or anti-solvent. In one embodiment, the compressed fluid and anti-solvent are both super critical carbon dioxide, and the critical temperature is at least 31.1°C and up to about 60°C, and the critical pressure is at least 1071 psi (7384272 Pa) and up to about 1800 psi (12410541 Pa).

In another embodiment, the compressed fluid and anti-solvent are both super critical carbon dioxide, and the critical temperature is at least 35°C and up to about 55°C, and the critical pressure is at least 1070 psi (7377377 Pa) and up to about 1500 psi (10342117 Pa) . It will be understood by those of skill in the art that the specific critical temperature and pressure may be different at different steps during the processing.

Any suitable pressurizable chamber may be used, including but not limited to those disclosed in WO2016/197091 or in U.S. Patent Nos. 5,833,891 and 5,874,029. Similarly, the steps of contacting the atomized droplets with the compressed fluid to cause depletion of the solvent from the droplets; and contacting the droplets with an anti-solvent to cause further depletion of the solvent from the droplets, to produce particles of the compound can be carried out under any suitable conditions, including but not limited to those disclosed in U.S. Patent Nos. 5,833,891 and 5,874,029.

The flow rate can be adjusted as high as possible to optimize output but below the pressure limitations for the equipment, including the nozzle orifice. In one embodiment, the flow rate of the solution through the nozzle has a range from about 0.5 mL/min to about 30 mL/min. In various further embodiments, the flow rate is between about 0.5 mL/min to about 25 mL/min, 0.5 mL/min to about 20 mL/min, 0.5 mL/min to about 15 mL/min, 0.5 mL/min to about 10 mL/min, 0.5 mL/min to about 4 mL/min, about 1 mL/min to about 30 mL/min, about 1 mL/min to about 25 mL/min, about 1 mL/min to about 20 mL/min, 1 mL/min to about 15 mL/min, about 1 mL/min to about 10 mL/min, about 2 mL/min to about 30 mL/min, about 2 mL/min to about 25 mL/min, about 2 mL/min to about 20 mL/min, about 2 mL/min to about 15 mL/min, or about 2 mL/min to about 10 mL/min. The solution of drug subject to the flow rate can be any suitable concentration, such as between about 1 mg/ml and about 80 mg/ml.

In one embodiment, the methods further comprise receiving the plurality of particles through the outlet of the pressurizable chamber; and collecting the plurality of particles in a collection device, such as disclosed in WO2016/197091.

In another aspect, the disclosure provides compound particles prepared by the method of any embodiment or combination of embodiments of the disclosure.

### Examples

### Methods

### Niclosamide

In one particular example method, a solution of 35 mg/ml of niclosamide was dissolved in a 1:1 acetone:ethanol (v:v) solution, or other solvent. The nozzle and a sonic probe were positioned in the pressurizable chamber approximately 9 mm apart. A sintered stainless steel mesh filter with approximately 20 nm holes was attached to the pressurizable chamber to collect the precipitated niclosamide nanoparticles. The supercritical carbon dioxide was placed in the pressurizable chamber of the manufacturing equipment and brought between 1085-1320 psi (7480798-9101063 Pa) at 35.0-42.6 °C and a flow rate of 4-12 kg per hour. The sonic probe was adjusted to an amplitude of 20-80% of maximum output at a frequency of 20 kHz. The 1:1 acetone: ethanol solution containing the niclosamide was pumped through the nozzle at a flow rate of 2 mL/minute for approximately 10 minutes. The precipitated niclosamide particles were then collected from the supercritical carbon dioxide as the mixture was pumped through the stainless steel mesh filter. The filter containing the nanoparticles of niclosamide was opened and the resulting product collected from the filter.

### Urea:Niclosamide Co-precipitation

In another particular example method, a solution of 20-25 mg/ml of 1:1 or 2:1 or 3:2 urea:niclosamide (mole:mole) was dissolved in a 1:1 acetone:ethanol (v:v) solution, or other solvent. The nozzle and a sonic probe were positioned in the pressurizable chamber approximately 9 mm apart. A sintered stainless steel mesh filter with approximately 20 nm holes was attached to the pressurizable chamber to collect the precipitated niclosamide nanoparticles. The supercritical carbon dioxide was placed in the pressurizable chamber of the manufacturing equipment and brought between 1090-1311 psi (7515272-9039010 Pa) at 35.5-42.3 °C and a flow rate of 4-12 kg per hour. The sonic probe was adjusted to an amplitude of 20-80% of maximum output at a frequency of 20 kHz. The 1:1 acetone:ethanol solution containing the urea:niclosamide was pumped through the nozzle at a flow rate of 2 mL/minute for approximately 15 minutes. The precipitated urea:niclosamide particles were then collected from the supercritical carbon dioxide as the mixture was pumped through the stainless steel mesh filter. The filter containing the nanoparticles of urea:niclosamide was opened and the resulting product collected from the filter.

### Nicotinamide:Niclosamide Co-precipitation

In another particular example method, a solution of 4 mg/ml of 1:1 nicotinamide:niclosamide (mole:mole) was dissolved in a 1:1 acetone:ethanol (v:v) solution, or other solvent. The nozzle and a sonic probe were positioned in the pressurizable chamber approximately 9 mm apart. A sintered stainless steel mesh filter with approximately 20 nm holes was attached to the pressurizable chamber to collect the precipitated nicotinamide:niclosamide nanoparticles. The supercritical carbon dioxide was placed in the pressurizable chamber of the manufacturing equipment and brought between 1190-1210 psi (8204746-8342641 Pa) at 37.2-37.8 °C and a flow rate of 4-12 kg per hour. The sonic probe was adjusted to an amplitude of 60% of maximum output at a frequency of 20 kHz. The 1:1 acetone:ethanol solution containing the nicotinamide:niclosamide was pumped through the nozzle at a flow rate of 2 mL/minute for approximately 100 minutes. The precipitated nicotinamide:niclosamide particles were then collected from the supercritical carbon dioxide as the mixture was pumped through the stainless steel mesh filter. The filter containing the nanoparticles of nicotinamide:niclosamide was opened and the resulting product collected from the filter.

### Niclosamide ethanolamine

In another particular example method, a solution of 12.5 mg/ml of niclosamide ethanolamine was dissolved in a 1:1 acetone:ethanol (v:v) solution. The nozzle and a sonic probe were positioned in the pressurizable chamber approximately 9 mm apart. A sintered stainless steel mesh filter with approximately 20 nm holes was attached to the pressurizable chamber to collect the precipitated niclosamide nanoparticles. The supercritical carbon dioxide was placed in the pressurizable chamber of the manufacturing equipment and brought between 1195-1210 psi (8239220-82805885 Pa) at 37.5-38.2 °C and a flow rate of 4-12 kg per hour. The sonic probe was adjusted to an amplitude of 60% of maximum output at a frequency of 20 kHz. The 1:1 acetone:ethanol solution containing the niclosamide ethanolamine was pumped through the nozzle at a flow rate of 2 mL/minute for approximately 50 minutes. The precipitated niclosamide ethanolamine particles were then collected from the supercritical carbon dioxide as the mixture was pumped through the stainless steel mesh filter. The filter containing the nanoparticles of niclosamide ethanolamine was opened and the resulting product collected from the filter.

Micronized niclosamide (supplied by Olon S.p.A. Italy) was prepared and used for comparison. Commonly, micronization is done by milling such as air jet milling or pin milling.

Niclosamide was evaluated for solubility in supercritical fluid carbon dioxide (scCO₂) and a variety of organic solvents. Approximately 63 precipitation runs (approximately 500mg to 10 grams) were conducted on the RC612B precipitation unit, according to examples provided above. Variables modified between each precipitation are detailed in Tables 1-3 below. The precipitates were analyzed by laser diffraction to determine PSD, BET sorptometry to determine SSA, and SEM to determine habit and support PSD and SSA data.

PSD analyses were conducted on a Malvern Mastersizer^{™} 3000 using the Aero S^{™} dispersion unit according to manufacturer's instructions.

SSA determination analyses were conducted on a Porous Materials, Inc., Automated BET Sorptometer BET-202A according to manufacturer's instructions.

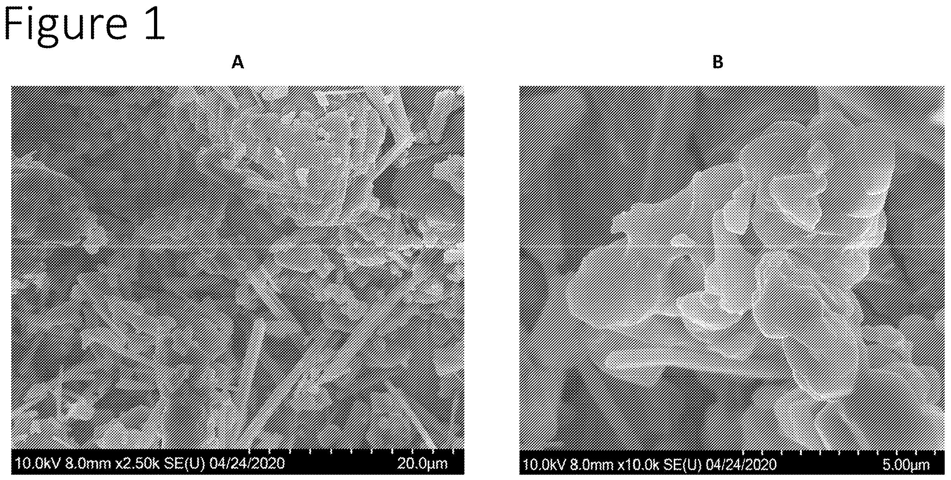

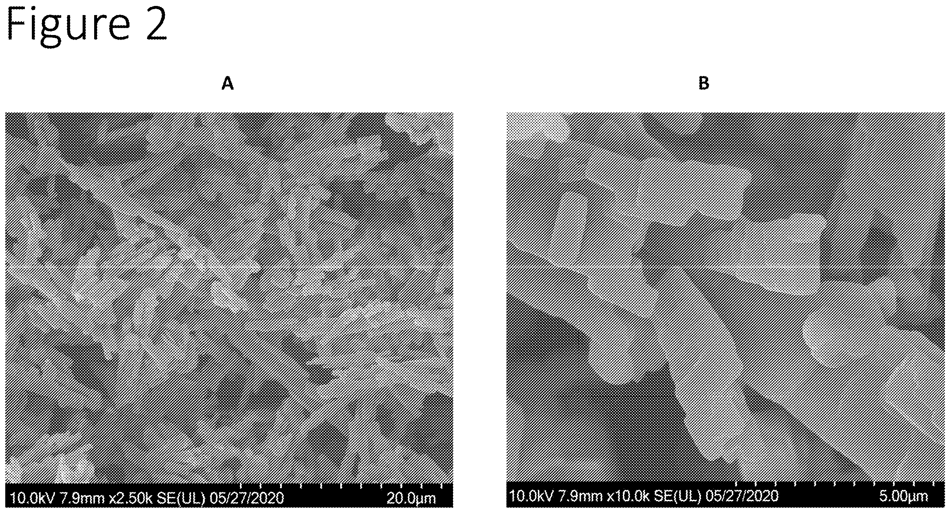

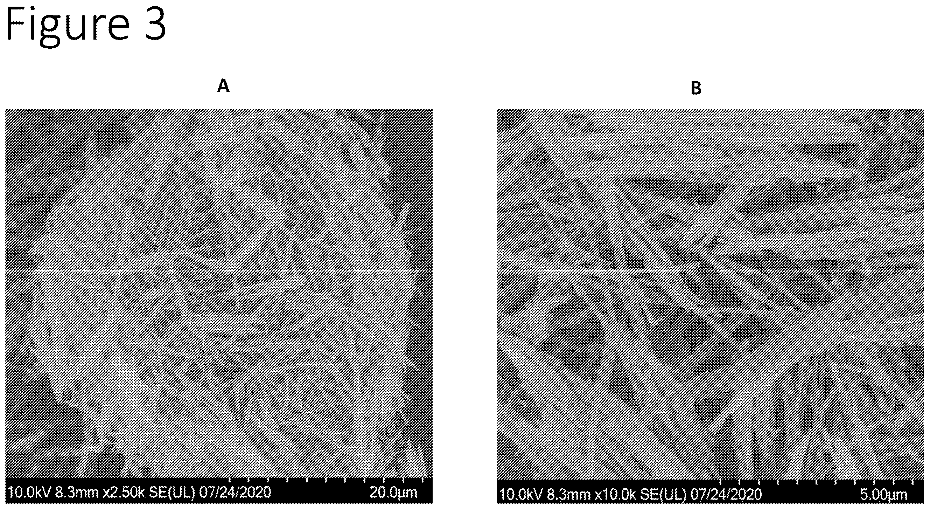

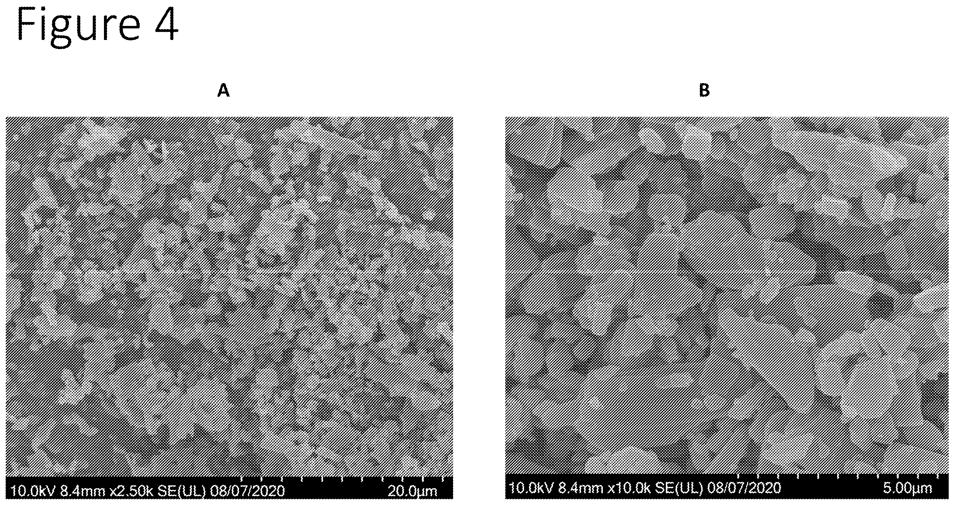

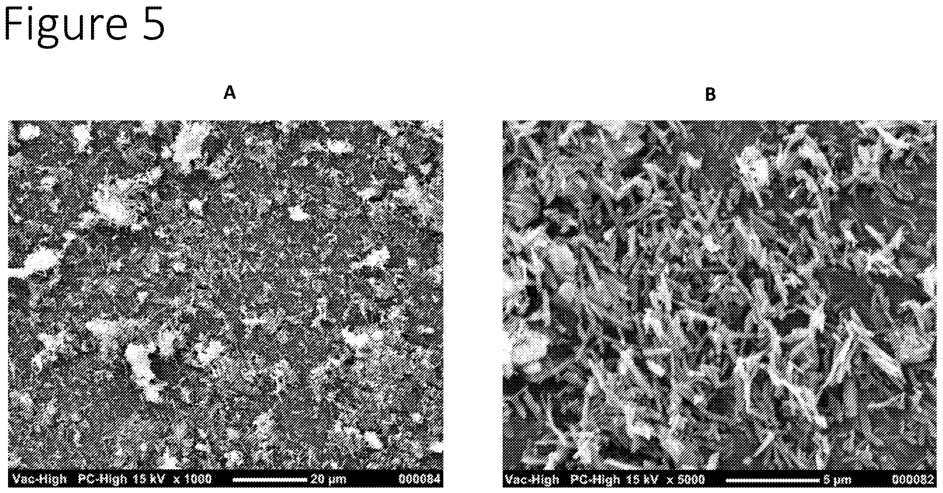

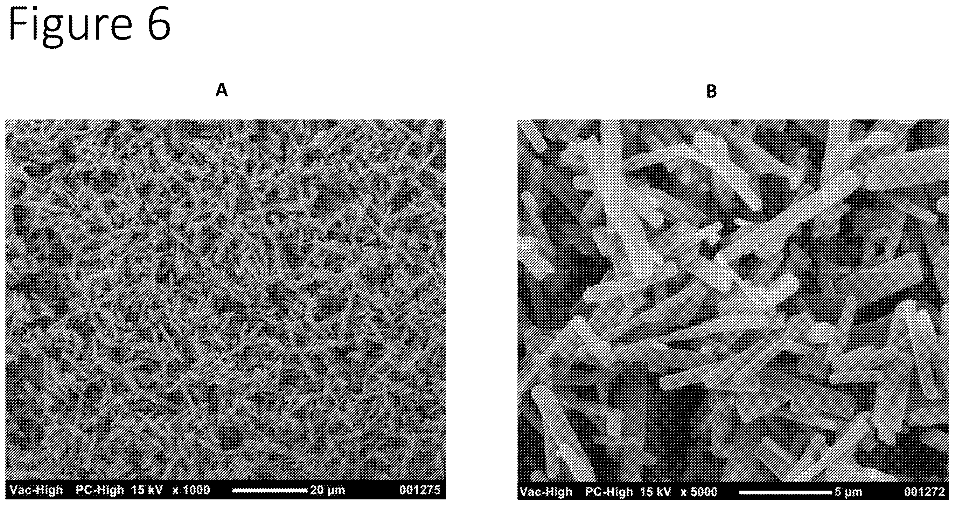

SEM was conducted on a Hitachi 8130 SEM. Exemplary SEM micrographs can be found in Figures 1-6.

Initial data are provided in Table 1-7. The tables provide general information on processing conditions, with "Low pressure" being 1190 to 1210 psi (8204746-8342641 Pa) "low temperature" being 20°C to 25°C, "high pressure" being 1300 to 1350 psi (8963168-9307906 Pa), "high temperature" being 40°C to 45° C, and "X" meaning average conditions between "high" and "low". These data demonstrate that the niclosamide particles produced have significantly reduced bulk density (tapped and untapped) and specific surface area compared to micronized niclosamide (see Table 2 for "micronized material" for the micronized niclosamide).

**Table 1. Niclosamide processing results precipitation runs 1-13**

| Drug: | Niclo sami de | | | | | | | | | | | | |
|---|---|---|---|---|---|---|---|---|---|---|---|---|---|
| Run # | 1 | 2 | 3 | 4 | 5 | 6 | 7 7 | 8 | 9 | 10 | 11 | 12 | 13 |
| Conditions | Solvent | | | | | Pressure | | Temperature | | Sonication | | Small Batch Production | |
| Solvent | Etha nol | Acet one | 1:1 Acet one: Etha nol | 1:1 Acet one: Meth anal | 1:1 Acet one: Etha nol | 1:1 Acet one: Etha nol | 1:1 Acet one: Etha nol | 1:1 Acet one: Etha nol | 1:1 Acet one: Etha nol | 1:1 Acet one: Etha nol | 1:1 Acet one: Etha nol | 1:1 Acet one: Etha nol | 1:1 Acet one: Etha nol |
| Concentrati on | 10 | 10 | 30 | 9 | 38 | 35 | 35 | 35 | 35 | 35 | 35 | 35 | 35 |
| Pressure | X | X | X | X | | Low | High | | X | X | X | X | X |
| Temperatur e | X | X | X | X | X | X | X | Low | High | X | X | High | X |
| scCO2 | X | X | X | Flowrate X | X X X X | X | X | | X | X | X | X | X |
| Sonication | X | X | X | X | X | X | X | X | X | Low | High | X | Low |
| Feed Rate | X | X | X | X | X | X | X | X | X | X | X | X | X |
| PSD - Mastersizer | | | | | | | | | | | | | |
| Dv-10 | 1.93 | 3.05 | 1.81 | 1.81 | 2.19 | 1.5 | 0.83 2 | 1.29 | 1.45 | 1.26 | 1.98 | 1.26 | 1.29 |
| Dv-50 | 8.41 | 8.6 | 6.32 | 6.87 | 6.37 | 4.87 | 2.61 | 4.28 | 4.38 | 4.63 | 5.34 | 5.72 | 4.44 |
| Dv-90 | 21.5 | 18.0 | 14.8 | 16.9 | 14.8 | 9.74 | 8.38 | 10.5 | 9.19 | 12.4 | 11.2 | 13.7 | 10.0 |
| SSA | | | | | | 4.61 | 6.86 | 4.87 4.87 4.87 48.7 | 3.90 | 6.05 | 7.25 | 5.57 | 7.47 |
| Bulk Density | | | | | | | | | | | | 0.18 8 | 0.11 3 |
| Tapped Density | | | | | | | | | | | | 0.28 2 | 0.15 9 |
| Yield | | | | | | | | | | | | | |
| Starting Mass | 0.613 | 0.601 2 | 0.60 01 | 0.623 9 | 0.60 74 | 0.70 5 | 0.70 18 | 0.69 59 | 0.69 54 | 0.70 67 | 0.70 99 | 5.01 18 | 5.03 81 |
| Mass Collected | 0.400 5 | 0.299 4 | 0.43 49 | 0.368 7 | 0.47 85 | 0.47 72 | 0.60 73 | 0.57 66 | 0.47 99 | 0.61 15 | 0.56 43 | 2.81 59 | 2.93 34 |
| % Yield | 65.29 % | 49.80 % | 72.4 7% | 59.10 % | 78.7 8% | 67.6 9% | 86.5 3% | 82.8 6% | 69.0 1% | 86.5 3% | 79.4 9% | 56.1 9% | 58.2 2% |

**Table 2. Niclosamide and initial co-precipitation processing results precipitation runs 14-20, and raw niclosamide (i.e.: "micronized material")**

| Drug: | Niclosa mide | | | | | | | |
|---|---|---|---|---|---|---|---|---|
| Run # Run # | 14 | 15 | 16 | 17 | 18 | 19 | 20 | NA |
| Conditio ns | Combination | | | 1:1 Co-precipitation (Urea:Niclos amide) | 2:1 Co-precipitation (Urea:Niclos amide) | 3:2 Co-precipitation (Urea:Niclos amide) | 1:1 Co-precipitation (Nicotinamide:Nic losamide) | Microni zed Niclosa mide API |
| Solvent | 1:1 Acetone | 1:1 Aceto | 1:1 Aceto | 1:1 Acetone: Ethanol | 1:1 Acetone: Ethanol | 1:1 Acetone: Ethanol | 1:1 Acetone: Ethanol | |
| | : Ethanol | ne: Ethan ol | ne: Ethan ol | | | | | |
| Concentr ation | 35 | 35 | 35 | 28 | 24 | 22.6 | 4.13 | |
| Pressure | Low | Low | X | X | X | X | X | |
| Temp | High | High | Low | X | X | X | X | |
| scCO2 Flowrate | X | X | X | X | X | X | X | |
| Sonicatio n | Low | X | Low | X | X | X | X | |
| Feed Rate | X | X | X | X | X | X | X | |
| PSD Mastersi zer | | | | | | | | |
| Dv-10 | 2.90 | 2.10 | 0.977 | 2.40 | 2.06 | 1.18 | 0.750 | 0.765 |
| Dv-50 | 9.49 | 6.20 | 3.66 | 7.50 | 8.09 | 6.38 | 2.67 | 2.97 |
| Dv-90 | 19.9 | 12.8 | 11.4 | 18.1 | 17.0 | 14.6 | 15.3 | 6.39 |
| SSA | | 1.95 | 3.43 | 7.04 | 7.32 | 5.24 | 13.87 | 3.26 |
| Bulk Density | 0.322 | 0.330 | 0.086 | 0.133 | 0.090 | 0.075 | 0.201 | 0.486 |
| Tapped Density | 0.515 | 0.462 | 0.112 | 0.240 | 0.098 | 0.089 | 0.241 | 0.619 |
| Yield | | | | | | | | |
| Starting Mass | 0.7057 | 0.700 1 | 0.7 | 0.8379 | 0.9659 | 0.904 | 0.8265 | |
| Mass Collecte d | 0.5793 | 0.560 5 | 0.636 4 | 0.4910 | 0.6792 | 0.5294 | 0.2375 | |
| % L Yield | 82.09% | 80.06 % | 90.91 % | 58.60% | 70.32% | 58.56% | 28.74% | |

**Table 3. Niclosamide co-precipitation 3:2 Urea:Niclosamide processing results precipitation runs 21-27**

| **Drug:** | **3:2 Urea:Nicl osamide** | | | | | | |
|---|---|---|---|---|---|---|---|
| **Run #** | **21** | **22** | **23** | **24** | **25** | **26** | **27** |
| **Conditions** | **Concentration** | **Pressure** | | **Temperature** | | **Sonication** | |
| Solvent | 1:1 Acetone: Ethanol | 1:1 Acetone: Ethanol | 1:1 Acetone: Ethanol | 1:1 Acetone: Ethanol | 1:1 Acetone: Ethanol | 1:1 Acetone: Ethanol | 1:1 Acetone: Ethanol |
| Concentration | 25 | 25 | 25 | 25 | 25 | 25 | 25 |
| Pressure | X | | High | X | X | X | X |
| Temperature | X | X | X | Low | High | X | X |
| scCO2 Flowrate | X | Low X | X | X | X | X | X |
| Sonication | X | X | X | X | X | Low | High |
| Feed Rate | X | X | X | X | X | X | X |

| **PSD** - **Mastersizer** | | | | | | | |
|---|---|---|---|---|---|---|---|
| Dv-10 | 2.30 | 1.14 | 2.04 | 2.37 | 1.07 | 1.71 | 1.90 |
| Dv-50 | 7.44 | 4.85 | 5.85 | 8.30 | 3.84 | 7.37 | 6.62 |
| Dv-90 | 15.9 | 9.82 | 12.1 | 17.8 | 7.92 | 16.9 | 14.2 |
| **SSA** | 7.136 | 8.151 | 6.043 | 5.379 | 6.146 | 10.563 | 10.154 |
| **Bulk Density** | 0.044 | 0.070 | 0.088 | 0.031 | 0.098 | 0.054 | 0.068 |

| **Tapped Density** | 0.059 | 0.087 | 0.111 | 0.037 | 0.133 | 0.077 | 0.088 |
|---|---|---|---|---|---|---|---|
| **Yield** | | | | | | | |
| Starting Mass | 0.6134 | 0.6012 | 0.6001 | 0.6239 | 0.6074 | 0.705 | 0.7018 |
| Mass Collected | 0.4005 | 0.2994 | 0.4349 | 0.3687 | 0.4785 | 0.4772 | 0.6073 |
| % Yield | 65.29% | 49.80% | 72.47% | 59.10% | 78.78% | 67.69% | 86.53% |

**Table 4. Niclosamide ethanolamine processing results precipitation runs 28-35**

| **Drug:** | **Niclosa mide ethanol amine** | | | | | | | | |
|---|---|---|---|---|---|---|---|---|---|
| **General Info** | | **SC2 8** | **SC29** | **SC30** | **SC31** | **SC32** | **SC33** | **SC34** | **SC35** |
| **Precipit ation Conditi ons** | | **Solvent/Concent ration** | | **Pressure** | | **Temperature** | | **Sonication** | |
| Solvent( s) | | Acet one | 1:1 Acetone: Ethanol | 1:1 Acetone: Ethanol | 1:1 Acetone: Ethanol | 1:1 Acetone: Ethanol | 1:1 Acetone: Ethanol | 1:1 Acetone: Ethanol | 1:1 Acetone: Ethanol |
| Concent ration (mg/mL ) | | 6.0 | 12.5 | 12.5 | 12.5 | 12.5 | 12.5 | 12.6 | 12.5 |
| Volume (mL) | | 100. 0 | 48.0 | 48.0 | 48.0 | 48.0 | 48.0 | 48.0 | 48.0 |
| Pressure | | X | X | High | Low | X | X | X | X |
| Temper ature | | X | X | X | X | High | Low | X | X |
| scCO2 Flowrat e | | X | X | X | X | X | X | X | X |
| Sonicati on | | X | X | X | X | X | X | X | X |
| Feed Rate | | X | X | X | X | X | X | Low | High |

| **PSD -** Masters izer | | **SC2 8** | **SC29** | **SC30** | **SC31** | **SC32** | **SC33** | **SC34** | **SC35** |
|---|---|---|---|---|---|---|---|---|---|
| D-10 (µm) | | 1.03 | 1.08 | 0.889 | 2.11 | 4.48 | 1.13 | 1.73 | 0.801 |
| D-50 (µm) | | 4.71 | 6.07 | 8.46 | 10.0 | 8.17 | 10.6 | 10.4 | 5.77 |
| D-90 (µm) | | 11.6 | 14.1 | 20.9 | 20.1 | 17.8 | 22.2 | 22.8 | 18.0 |
| SSA (m²/g) | 2.684 | 8.88 4 | 11.971 | 28.727 | 29.823 | 18.582 | 17.075 | 24.949 | 19.228 |
| **Bulk Density** (g/mL) | | 0.08 29 | 0.0575 | 0.075 | 0.069 | 0.056 | 0.079 | 0.069 | 0.064 |
| **Tapped Density** (g/mL) | | 0.09 05 | 0.0600 | 0.084 | 0.084 | 0.073 | 0.099 | 0.079 | 0.079 |

| **Yield** | | **SC2 8** | **SC29** | **SC30** | **SC31** | **SC32** | **SC33** | **SC34** | **SC35** |
|---|---|---|---|---|---|---|---|---|---|
| Starting Mass (g) | | 0.59 91 | 0.6007 | 0.6015 | 0.5992 | 0.6006 | 0.5995 | 0.6054 | 0.5999 |
| Mass Collecte d (g) | | 0.49 15 | 0.4983 | 0.4873 | 0.4252 | 0.4640 | 0.5205 | 0.4674 | 0.4917 |
| % Yield | | 82.0 4% | 82.95% | 81.01% | 70.96% | 77.26% | 86.82% | 77.21% | 81.96% |

**Table 5. Niclosamide processing results precipitation runs 36-44**

| **Drug:** | | **Niclos amide** | | | | | | | | | |
|---|---|---|---|---|---|---|---|---|---|---|---|
| **General Info** | | | **SC36** | **SC37** | **SC38** | **SC39** | **SC40** | **SC41** | **SC4 2** | **SC 43** | **SC 44** |
| **Precipitatio n Conditions** | | | **Combination** | | | | | | **Solvents** | | |
| Solvent(s) | | | 1:1 Acetone: Ethanol | 1:1 Acetone: Ethanol | 1:1 Acetone: Ethanol | 1:1 Acetone: Ethanol | 1:1 Acetone: Ethanol | 1:1 Acetone: Ethanol | NM P | ME K | TH F |
| Concentratio n(mg/mL) | | | 35.0 | 35.0 | 35.0 | 35.0 | 35.0 | 35.0 | 20.0 | 10.0 | 35.0 |
| Volume (mL) | | | 20.0 | 20.0 | 20.0 | 20.0 | 20.0 | 20.0 | 35.0 | 70.0 | 20.0 |
| Pressure | | | X | X | High | High | X | High | X | X | X |
| Temperature | | | X | X | X | X | X | X | X | X | X |
| scCO2 Flowrate | | | High | Low | X | Low | X | X | X | X | X |
| Sonication | | | Low | Low | Low | Low | None | Low | X | X | X |
| Feed Rate | | | X | X | X | X | X | X | X | X | X |
| PSD - **Mastersizer** | | | SC36 | SC37 | **SC38** | **SC39** | **SC40** | **SC41** | SC4 2 | SC 43 | SC **44** |
| | D-10 (µm) | | 3.23 | 1.02 | 0.898 | 1.03 | 2.00 | 0.970 | 3.15 | 3.33 0 | 1.09 |
| | D-50 (µm) | | 7.35 | 3.57 | 2.83 | 3.39 | 6.07 | 3.79 | 7.42 | 8.22 | 4.26 |
| | D-90 (µm) | | 14.7 | 12.7 | 8.55 | 9.32 | 16.5 | 12.1 | 15.9 | 17.2 | 8.58 |
| **SSA** (m²/g) | | | 8.981 | 5.486 | 5.472 | 8.367 | 6.125 | | 3.09 | 5.60 | 12.7 6 |
| **Bulk Density** (g/mL) | | | | | | | | | 0.09 2 | 0.07 0 | |
| **Tapped Density** (g/mL) | | | | | | | | | 0.14 0 | 0.09 8 | |
| **Yield** | | | **SC36** | **SC37** | **SC38** | **SC39** | **SC40** | **SC41** | **SC4 2** | **SC 43** | **SC 44** |
| Starting Mass (g) | | | 0.6981 | 0.699 | 0.7011 | 0.6958 | 0.7061 | 0.6949 | 0.70 6 | 0.70 4 | 0.69 91 |
| Mass Collected (g) | | | 0.5052 | 0.5338 | 0.6059 | 0.5708 | 0.4822 | 0.3574 | 0.72 83 | 0.40 79 | 0.44 07 |
| | % Yield | | 72.37% | 76.37% | 86.42% | 82.04% | 68.29% | 51.43% | 103. 16% | 57.9 4% | 63.0 4% |

**Table 6. Niclosamide processing results precipitation runs 45-54**

| **Drug:** | **Niclosamid e** | | | | | | | | | |
|---|---|---|---|---|---|---|---|---|---|---|
| **Run** # | 45 | 46 | 47 | 48 | 49 | 50 | 51 | 52 | 53 | 54 |
| **Conditions** | **Pressure** | | **Temperature** | | **Flow** | **Sonication** | | | **Combination** | |
| Solvent | THF | THF | THF | THF | THF | THF | THF | THF | THF | THF |
| Concentratio n | 35 | 35 | 35 | 35 | 35 | 35 | 35 | 35 | 35 | 35 |
| Pressure | High | Low | X | X | X | X | X | X | High | X |
| Temperature | X | X | High | Low | X | X | X | X | Low | Low |
| scCO2 Flowrate | X | X | X | X | High | X | X | X | X | X |
| Sonication | X | X | X | X | X | High | Low | None | Low | Low |
| Feed Rate | X | X | X | X | X | X | X | X | X | X |
| **PSD** - **Mastersizer** | | | | | | | | | | |
| Dv-10 | 0.769 | 2.82 | 3.77 | 2.51 | 1.28 | 1.77 | 1.19 | 1.06 | 0.991 | 0.928 |
| Dv-50 | 2.28 | 6.89 | 8.82 | 6.32 | 4.14 | 4.58 | 4.85 | 3.77 | 4.13 | 4.01 |
| Dv-90 | 6.36 | 12.5 | 16.3 | 12.6 | 8.30 | 9.37 | 10.60 | 8.97 | 11.7 | 10.8 |
| **SSA** | 12.758 | 8.159 | 5.008 | 4.722 | 6.530 | 5.548 | 7.580 | 5.673 | 7.846 | 10.674 |
| **Yield** | | | | | | | | | | |
| Starting Mass | 0.6978 | 0.6991 | 0.694 | 0.7011 | 0.7018 | 0.6958 | 0.696 | 0.6989 | 0.7078 | 0.6997 |
| Mass Collected | 0.5414 | 0.5757 | 0.5914 | 0.4467 | 0.5513 | 0.5049 | 0.5437 | 0.5587 | 0.5615 | 0.4115 |
| % Yield | 77.59% | 82.35 % | 85.22 % | 63.71 % | 78.56 % | 72.56 % | 78.12 % | 79.94 % | 79.33 % | 58.81 % |

**Table 7. Niclosamide processing results precipitation runs 55-63**

| **Drug:** | **Niclosami de** | | | | | | | | |
|---|---|---|---|---|---|---|---|---|---|
| **Run** # | 55 | 56 | 57 | 58 | 59 | 60 | 61 | 62 | 63 |

| **Conditions** | **Standard** | | **10g** | | | **Solvent** | | | |
|---|---|---|---|---|---|---|---|---|---|
| Solvent | THF | THF | 1:1 Aceton e: Ethano l | THF | THF | 75:25 THF:Etha nol | 75:25 THF:Aceto ne | 1:1 THF:Metha nol | 75:25 THF:Etha nol |
| Concentrati on | 35 | 35 | 35 | 35 | 35 | 50 | 35 | 15 | 50 |
| Pressure | X | X | High | X | X | X | X | X | X |
| Temperatur e | X | X | X | Low | Low | X | X | X | Low |
| scCO2 Flowrate | X | X | Low | X | X | X | X | X | X |
| Sonication | X | X | Low | Low | Low | X | X | X | Low |
| Feed Rate | X | X | X | X | X | X | X | X | X |

| **PSD - Mastersize r** | | | | | | | | | |
|---|---|---|---|---|---|---|---|---|---|
| Dv-10 | 2.43 | 1.59 | 1.59 | 1.07 | 1.09 | 1.46 | 2.37 | 2.89 | 0.984 |
| Dv-50 | 6.2 | 4.98 | 5.93 | 4.92 | 5.40 | 4.40 | 6.57 | 9.01 | 3.69 |
| Dv-90 | 12.2 | 11.2 | 14.5 | 13.0 | 14.6 | 9.68 | 13.3 | 20.0 | 10.6 |
| **SSA** | 3.910 | 3.668 | 2.416 | 6.931 | 4.23 | 4.662 | 5.104 | 4.025 | 6.474 |

| **Yield** | | | | | | | | | |
|---|---|---|---|---|---|---|---|---|---|
| Starting Mass | 0.7072 | 0.703 1 | 10.002 3 | 10.00 8 | 10.00 39 | 0.6996 | 0.6945 | 0.7038 | 0.7012 |
| Mass Collected | 0.5234 | 0.499 | 6.84 | 9.05 | 8.33 | 0.4469 | 0.4819 | 0.4506 | 0.4152 |
| % Yield | 74.01% | 70.97 % | 68.38 % | 90.43 % | 83.27 % | 63.88% | 63.39% | 64.02% | 59.21% |

Exemplary processed products were also used for dissolution for comparison to unprocessed/raw material. Dissolution studies were carried out in 40% isopropanol 37°C with a rotating paddle at 50 rpm. Data are provided in Table 8, showing a significant improvement in dissolution properties compared to raw niclosamide and micronized niclosamide.

Mass median aerodynamic diameter (MMAD) properties of exemplary aerosol droplets of processed products were also determined using a rotating brush generator and a TSI Aerodymanic Particle Sizer, Model 3321. Data are provided in Table 8.

**Table 8. MMAD and dissolution Data**

| | Niclosam ide Drug Substanc e | Milled (microniz ed) Niclosam ide Drug Substance | SC13 SCP Processed Niclosamide from Acetone:Eth anol solvent | SC58 SCP Processed Niclosam ide from THF solvent | SC27 SCP Processed Niclosamide: Urea Cocrystal from Acetone:Etha nol | SC31 SCP Processed Niclosamide:ethanol amine from Acetone:Ethanol |
|---|---|---|---|---|---|---|
| Mean Geometric Particle size Dv50 (microns) | 21.1 | 2.97 | 4.44 | 4.92 | 6.62 | 10 |
| MMAD / GSD (microns) | 2.64 / 1.82 | 2.26 / 1.74 | 2.69 / 1.63 | 2.00 / 1.47 | 1.96 /1.60 | 2.90 /1.85 |
| Bulk Density (gm/cm3) | 0.45 | 0.49 | 0.113 | 0.0937 | 0.068 | 0.069 |
| Bulk Tapped Density (gm/cm3) | 0.57 | 0.62 | 0.159 | 0.115 | 0.088 | 0.084 |
| Surface Area (m2/gm) | 1.359 | 3.264 | 7.47 | 6.931 | 10.154 | 29.82 |

| Dissolution in 40% isopropanol 37C 50rpm rotating paddle (%) | | | | | | |
|---|---|---|---|---|---|---|
| 5 min | 1.7 | 2.9 | 27.7 | 6.1 | 6.2 | 27.1 |
| 15 min | 3.8 | 4.7 | 28.4 | 8.8 | 9.2 | 42.1 |
| 30 min | 6.5 | 8.4 | 30.7 | 13.1 | 16.3 | 50.9 |
| 60 min | 11.2 | 12.7 | 35.1 | 18.3 | 21.1 | 55.8 |
| 90 min | 15 | 17.3 | 38.2 | 24.4 | 27.4 | 56 |
| 120 min | 20.6 | 23.3 | 41.1 | 31.3 | 40 | 67.5 |

A pharmacokinetic study will be conducted in adult Sprague-Dawley rats comparing a raw or micronized niclosamide suspension administered by oral gavage to exemplary niclosamide particles/compositions of the disclosure ('SCP Niclosamide") administered by inhalation using nose only inhalation. The SCP niclosamide will be aerosolized using a rotating brush generator and the air flow will be adjusted to provide a constant and consistent amount of niclosamide in the inhalation air. Samples of the inhalation air will be periodically assayed to assure consistent dosing.

Each group of 24 rats plus extras will be administered a single dose of the test article at time zero. At each of 8 time points, 3 rats will be sacrificed from each dosing group and samples of the blood and lung tissue will be collected. Example time points beyond Time Zero are 1, 2, 4, 8, 24, 48, 72 and 96 hours after dosing. Blood samples will be kept in an ice bath until they can be centrifuged to allow for separation and collection of the plasma. Lung tissue and plasma samples will be frozen at -20°C until they are thawed prior to assay. Samples will be assayed using a validated LCMSMS assay. The data will be evaluated using a non-compartmental analysis to determine Cmax, Tmax, AUC and the apparent terminal half-life for niclosamide from the plasma and lung tissue.

### Reference List

Ai, N., Wood, R.D., Yang, E., and Welsh, W.J. (2016). Niclosamide is a Negative Allosteric Modulator of Group I Metabotropic Glutamate Receptors: Implications for Neuropathic Pain. Pharm Res 33, 3044-3056.
Andrews, P., Thyssen, J., and Lorke, D. (1982). The biology and toxicology of molluscicides, Bayluscide. Pharmacol Ther 19, 245-295.
Benedetto, R., Cabrita, I., Schreiber, R., and Kunzelmann, K. (2019). TMEM16A is indispensable for basal mucus secretion in airways and intestine. FASEB journal : official publication of the Federation of American Societies for Experimental Biology 33, 4502-4512. Berube, B.J., Castro, L., Russell, D., Ovechkina, Y., and Parish, T. (2018). Novel Screen to Assess Bactericidal Activity of Compounds Against Non-replicating Mycobacterium abscessus. Front Microbiol 9, 2417.
Boyapally, R., Pulivendala, G., Bale, S., and Godugu, C. (2019). Niclosamide alleviates pulmonary fibrosis in vitro and in vivo by attenuation of epithelial-to-mesenchymal transition, matrix proteins & Wnt/beta-catenin signaling: A drug repurposing study. Life sciences 220, 8-20.
Cabrita, I., Benedetto, R., Schreiber, R., and Kunzelmann, K. (2019). Niclosamide repurposed for the treatment of inflammatory airway disease. JCI Insight 4.
Cabrita, I., Buchholz, B., Schreiber, R., and Kunzelmann, K. (2020a). TMEM16A drives renal cyst growth by augmenting Ca(2+) signaling in M1 cells. J Mol Med (Berl) 98, 659-671.
Cabrita, I., Kraus, A., Scholz, J.K., Skoczynski, K., Schreiber, R., Kunzelmann, K., and Buchholz, B. (2020b). Cyst growth in ADPKD is prevented by pharmacological and genetic inhibition of TMEM16A in vivo. Nature communications 11, 4320.
Centeio, R., Cabrita, I., Benedetto, R., Talbi, K., Ousingsawat, J., Schreiber, R., Sullivan, J.K., and Kunzelmann, K. (2020). Pharmacological Inhibition and Activation of the Ca(2+) Activated Cl(-) Channel TMEM16A. International journal of molecular sciences 21.
Chang, X., Zhen, X., Liu, J., Ren, X., Hu, Z., Zhou, Z., Zhu, F., Ding, K., and Nie, J. (2017). The antihelmenthic phosphate niclosamide impedes renal fibrosis by inhibiting homeodomain-interacting protein kinase 2 expression. Kidney international.
Chen, W., Mook, R.A., Jr., Premont, R.T., and Wang, J. (2017). Niclosamide: Beyond an antihelminthic drug. Cell Signal.
Cho, H., Yang, Y.D., Lee, J., Lee, B., Kim, T., Jang, Y., Back, S.K., Na, H.S., Harfe, B.D., Wang, F., et al. (2012). The calcium-activated chloride channel anoctamin 1 acts as a heat sensor in nociceptive neurons. Nature neuroscience 15, 1015-1021.
Chowdhury, M.K., Turner, N., Bentley, N.L., Das, A., Wu, L.E., Richani, D., Bustamante, S., Gilchrist, R.B., Morris, M.J., Shepherd, P.R., et al. (2017). Niclosamide reduces glucagon sensitivity via hepatic PKA inhibition in obese mice: Implications for glucose metabolism improvements in type 2 diabetes. Scientific reports 7, 40159.
Copp, J.N., Pletzer, D., Brown, A.S., Van der Heijden, J., Miton, C.M., Edgar, R.J., Rich, M.H., Little, R.F., Williams, E.M., Hancock, R.E.W., et al. (2020). Mechanistic understanding enables the rational design of salicylanilide combination therapies for Gram-negative infections. bioRxiv, 2020.2004.2023.058875.
Danielsson, J., Vink, J., Hyuga, S., Fu, X.W., Funayama, H., Wapner, R., Blanks, A.M., and Gallos, G. (2018). Anoctamin Channels in Human Myometrium: A Novel Target for Tocolysis. Reprod Sci, 1933719118757683.
Du, Y.H., and Guan, Y.Y. (2015). Chloride Channels - New Targets for the Prevention of Stroke. Curr Vasc Pharmacol 13, 441-448.
Fan, X., Xu, J., Files, M., Cirillo, J.D., Endsley, J.J., Zhou, J., and Endsley, M.A. (2019). Dual activity of niclosamide to suppress replication of integrated HIV-1 and Mycobacterium tuberculosis (Beijing). Tuberculosis (Edinburgh, Scotland) 116s, S28-s33.
Forrest, A.S., Joyce, T.C., Huebner, M.L., Ayon, R.J., Wiwchar, M., Joyce, J., Freitas, N., Davis, A.J., Ye, L., Duan, D.D., et al. (2012). Increased TMEM16A-encoded calcium-activated chloride channel activity is associated with pulmonary hypertension. American journal of physiology Cell physiology 303, C1229-1243.
Gassen, N.C., Niemeyer, D., Muth, D., Corman, V.M., Martinelli, S., Gassen, A., Hafner, K., Papies, J., Mosbauer, K., Zellner, A., et al. (2019). SKP2 attenuates autophagy through Beclin1-ubiquitination and its inhibition reduces MERS-Coronavirus infection. Nature communications 10, 5770.
Gassen, N.C., Papies, J., Bajaj, T., Dethloff, F., Emanuel, J., Weckmann, K., Heinz, D.E., Heinemann, N., Lennarz, M., Richter, A., et al. (2020). Analysis of SARS-CoV-2-controlled autophagy reveals spermidine, MK-2206, and niclosamide as putative antiviral therapeutics. bioRxiv, 2020.2004.2015.997254.
Han, P., Zhan, H., Shao, M., Wang, W., Song, G., Yu, X., Zhang, C., Ge, N., Yi, T., Li, S., et al. (2018). Niclosamide ethanolamine improves kidney injury in db/db mice. Diabetes Res Clin Pract 144, 25-33.
Heinze, C., Seniuk, A., Sokolov, M.V., Huebner, A.K., Klementowicz, A.E., Szijarto, I.A., Schleifenbaum, J., Vitzthum, H., Gollasch, M., Ehmke, H., et al. (2014). Disruption of vascular Ca2+-activated chloride currents lowers blood pressure. J Clin Invest 124, 675-686. Hulseberg, C.E., Feneant, L., Szymanska-de Wijs, K.M., Kessler, N.P., Nelson, E.A., Shoemaker, C.J., Schmaljohn, C.S., Polyak, S.J., and White, J.M. (2019). Arbidol and Other Low-Molecular-Weight Drugs That Inhibit Lassa and Ebola Viruses. J Virol 93.
Imperi, F., Massai, F., Ramachandran Pillai, C., Longo, F., Zennaro, E., Rampioni, G., Visca, P., and Leoni, L. (2013). New life for an old drug: the anthelmintic drug niclosamide inhibits Pseudomonas aeruginosa quorum sensing. Antimicrobial agents and chemotherapy 57, 996-1005.
Jeon, S., Ko, M., Lee, J., Choi, I., Byun, S.Y., Park, S., Shum, D., and Kim, S. (2020). Identification of antiviral drug candidates against SARS-CoV-2 from FDA-approved drugs. bioRxiv, 2020.2003.2020.999730.
Jurgeit, A., McDowell, R., Moese, S., Meldrum, E., Schwendener, R., and Greber, U.F. (2012). Niclosamide is a proton carrier and targets acidic endosomes with broad antiviral effects. PLoS pathogens 8, e1002976.
Kao, J.C., HuangFu, W.C., Tsai, T.T., Ho, M.R., Jhan, M.K., Shen, T.J., Tseng, P.C., Wang, Y.T., and Lin, C.F. (2018). The antiparasitic drug niclosamide inhibits dengue virus infection by interfering with endosomal acidification independent of mTOR. PLoS neglected tropical diseases 12, e0006715.
Lambert, M., Capuano, V., Olschewski, A., Sabourin, J., Nagaraj, C., Girerd, B., Weatherald, J., Humbert, M., and Antigny, F. (2018). Ion Channels in Pulmonary Hypertension: A Therapeutic Interest? International journal of molecular sciences 19*.*
Leblanc, N., Forrest, A.S., Ayon, R.J., Wiwchar, M., Angermann, J.E., Pritchard, H.A., Singer, C.A., Valencik, M.L., Britton, F., and Greenwood, I.A. (2015). Molecular and functional significance of Ca(2+)-activated Cl(-) channels in pulmonary arterial smooth muscle. Pulm Circ 5, 244-268.
Li, S.L., Yan, J., Zhang, Y.Q., Zhen, C.L., Liu, M.Y., Jin, J., Gao, J.L., Xiao, X.L., Shen, X., Tai, Y., et al. (2017a). Niclosamide ethanolamine inhibits artery constriction. Pharmacological research 115, 78-86.
Li, Z., Brecher, M., Deng, Y.Q., Zhang, J., Sakamuru, S., Liu, B., Huang, R., Koetzner, C.A., Allen, C.A., Jones, S.A., et al. (2017b). Existing drugs as broad-spectrum and potent inhibitors for Zika virus by targeting NS2B-NS3 interaction. Cell Res 27, 1046-1064.
Liang, L., Huang, M., Xiao, Y., Zen, S., Lao, M., Zou, Y., Shi, M., Yang, X., and Xu, H. (2015). Inhibitory effects of niclosamide on inflammation and migration of fibroblast-like synoviocytes from patients with rheumatoid arthritis. Inflamm Res 64, 225-233.
Liu, P.Y., Zhang, Z., Liu, Y., Tang, X.L., Shu, S., Bao, X.Y., Zhang, Y., Gu, Y., Xu, Y., and Cao, X. (2019). TMEM16A Inhibition Preserves Blood-Brain Barrier Integrity After Ischemic Stroke. Frontiers in cellular neuroscience 13, 360.
Ma, M.M., Gao, M., Guo, K.M., Wang, M., Li, X.Y., Zeng, X.L., Sun, L., Lv, X.F., Du, Y.H., Wang, G.L., et al. (2017). TMEM16A Contributes to Endothelial Dysfunction by Facilitating Nox2 NADPH Oxidase-Derived Reactive Oxygen Species Generation in Hypertension. Hypertension.
Mazzon, M., Ortega-Prieto, A.M., Imrie, D., Luft, C., Hess, L., Czieso, S., Grove, J., Skelton, J.K., Farleigh, L., Bugert, J.J., et al. (2019). Identification of Broad-Spectrum Antiviral Compounds by Targeting Viral Entry. Viruses 11.
Miner, K., Labitzke, K., Liu, B., Wang, P., Henckels, K., Gaida, K., Elliott, R., Chen, J.J., Liu, L., Leith, A., et al. (2019). Drug Repurposing: The Anthelmintics Niclosamide and Nitazoxanide Are Potent TMEM16A Antagonists That Fully Bronchodilate Airways. Front Pharmacol 10, 51.
Mohammad, H., AbdelKhalek, A., Abutaleb, N.S., and Seleem, M.N. (2018). Repurposing niclosamide for intestinal decolonization of vancomycin-resistant enterococci. Int J Antimicrob Agents 51, 897-904.
Morin, F., Kavian, N., Nicco, C., Cerles, O., Chereau, C., and Batteux, F. (2016). Improvement of Sclerodermatous Graft-Versus-Host Disease in Mice by Niclosamide. J Invest Dermatol 136, 2158-2167.
Namkung, W., Thiagarajah, J.R., Phuan, P.W., and Verkman, A.S. (2010). Inhibition of Ca2+-activated Cl- channels by gallotannins as a possible molecular basis for health benefits of red wine and green tea. FASEB journal : official publication of the Federation of American Societies for Experimental Biology 24, 4178-4186.
Ousingsawat, J., Wanitchakool, P., Schreiber, R., and Kunzelmann, K. (2018). Contribution of TMEM16F to pyroptotic cell death. Cell death & disease 9, 300*.*
Papp, R., Nagaraj, C., Zabini, D., Nagy, B.M., Lengyel, M., Skofic Maurer, D., Sharma, N., Egemnazarov, B., Kovacs, G., Kwapiszewska, G., et al. (2019). Targeting TMEM16A to reverse vasoconstriction and remodelling in idiopathic pulmonary arterial hypertension. Eur Respir J 53*.*
Paraskevopoulos, G., Kratky, M., Mandikova, J., Trejtnar, F., Stolarikova, J., Pavek, P., Besra, G., and Vinsova, J. (2015). Novel derivatives of nitro-substituted salicylic acids: Synthesis, antimicrobial activity and cytotoxicity. Bioorganic & medicinal chemistry 23, 7292-7301.
Prather, G.R., MacLean, J.A., 2nd, Shi, M., Boadu, D.K., Paquet, M., and Hayashi, K. (2016). Niclosamide As a Potential Nonsteroidal Therapy for Endometriosis That Preserves Reproductive Function in an Experimental Mouse Model. Biology of reproduction.
Rajamuthiah, R., Fuchs, B.B., Conery, A.L., Kim, W., Jayamani, E., Kwon, B., Ausubel, F.M., and Mylonakis, E. (2015). Repurposing salicylanilide anthelmintic drugs to combat drug resistant Staphylococcus aureus. PloS one 10, e0124595.
Ramsey, K.A., Chen, A.C.H., Radicioni, G., Lourie, R., Martin, M., Broomfield, A., Sheng, Y.H., Hasnain, S.Z., Radford-Smith, G., Simms, L.A., et al. (2020). Airway Mucus Hyperconcentration in Non-Cystic Fibrosis Bronchiectasis. Am J Respir Crit Care Med 201, 661-670.
Schweizer, M.T., Haugk, K., McKiernan, J.S., Gulati, R., Cheng, H.H., Maes, J.L., Dumpit, R.F., Nelson, P.S., Montgomery, B., McCune, J.S., et al. (2018). A phase I study of niclosamide in combination with enzalutamide in men with castration-resistant prostate cancer. PloS one 13, e0198389.
Sekulovski, N., Whorton, A.E., Tanaka, T., Hirota, Y., Shi, M., MacLean, J.A., de Mola, J.R.L., Groesch, K., Diaz-Sylvester, P., Wilson, T., et al. (2020). Niclosamide suppresses macrophage-induced inflammation in endometriosis. Biology of reproduction 102, 1011-1019.
Singh, H., Singh, A.K., Sharma, S., Iyer, R.N., and Srivastava, O.P. (1977). Synthesis of 5-chloro-3'-nitro-4'-substituted salicylanilides, a new series of anthelmintic and antimicrobial agents. J Med Chem 20, 826-829.
Stachulski, A.V., Pidathala, C., Row, E.C., Sharma, R., Berry, N.G., Lawrenson, A.S., Moores, S.L., Iqbal, M., Bentley, J., Allman, S.A., et al. (2011). Thiazolides as novel antiviral agents. 2. Inhibition of hepatitis C virus replication. J Med Chem 54, 8670-8680. Sun, Z., and Zhang, Y. (1999). Antituberculosis activity of certain antifungal and antihelmintic drugs. Tuber Lung Dis 79, 319-320.
Takayama, Y., Uta, D., Furue, H., and Tominaga, M. (2015). Pain-enhancing mechanism through interaction between TRPV1 and anoctamin 1 in sensory neurons. Proceedings of the National Academy of Sciences of the United States of America 112, 5213-5218.
Tang, D., Comish, P., and Kang, R. (2020). The hallmarks of COVID-19 disease. PLoS pathogens 16, e1008536.
Tao, H., Zhang, Y., Zeng, X., Shulman, G.I., and Jin, S. (2014). Niclosamide ethanolamine-induced mild mitochondrial uncoupling improves diabetic symptoms in mice. Nature medicine 20, 1263-1269.
Thatikonda, S., Pooladanda, V., and Godugu, C. (2020). Repurposing an old drug for new use: Niclosamide in psoriasis-like skin inflammation. Journal of cellular physiology 235, 5270-5283.
Thiagarajah, J.R., Ko, E.A., Tradtrantip, L., Donowitz, M., and Verkman, A.S. (2014). Discovery and development of antisecretory drugs for treating diarrheal diseases. Clin Gastroenterol Hepatol 12, 204-209.
Torres, N.S., Abercrombie, J.J., Srinivasan, A., Lopez-Ribot, J.L., Ramasubramanian, A.K., and Leung, K.P. (2016). Screening a Commercial Library of Pharmacologically Active Small Molecules against Staphylococcus aureus Biofilms. Antimicrobial agents and chemotherapy 60, 5663-5672.
van Tonder, E.C., Mahlatji, M.D., Malan, S.F., Liebenberg, W., Caira, M.R., Song, M., and de Villiers, M.M. (2004). Preparation and physicochemical characterization of 5 niclosamide solvates and 1 hemisolvate. AAPS PharmSciTech 5, E12.
Vanoni, S., Zeng, C., Marella, S., Uddin, J., Wu, D., Arora, K., Ptaschinski, C., Que, J., Noah, T., Waggoner, L., et al. (2020). Identification of anoctamin 1 (ANO1) as a key driver of esophageal epithelial proliferation in eosinophilic esophagitis. J Allergy Clin Immunol 145, 239-254 e232.
Wang, Y.M., Lu, J.W., Lin, C.C., Chin, Y.F., Wu, T.Y., Lin, L.I., Lai, Z.Z., Kuo, S.C., and Ho, Y.J. (2016). Antiviral activities of niclosamide and nitazoxanide against chikungunya virus entry and transmission. Antiviral Res 135, 81-90.
Wen, C.C., Kuo, Y.H., Jan, J.T., Liang, P.H., Wang, S.Y., Liu, H.G., Lee, C.K., Chang, S.T., Kuo, C.J., Lee, S. S., et al. (2007). Specific plant terpenoids and lignoids possess potent antiviral activities against severe acute respiratory syndrome coronavirus. J Med Chem 50, 4087-4095.
Wu, C.J., Jan, J.T., Chen, C.M., Hsieh, H.P., Hwang, D.R., Liu, H.W., Liu, C.Y., Huang, H.W., Chen, S.C., Hong, C.F., et al. (2004). Inhibition of severe acute respiratory syndrome coronavirus replication by niclosamide. Antimicrobial agents and chemotherapy 48, 2693-2696.
Xu, M., Lee, E.M., Wen, Z., Cheng, Y., Huang, W.K., Qian, X., Tcw, J., Kouznetsova, J., Ogden, S.C., Hammack, C., et al. (2016). Identification of small-molecule inhibitors of Zika virus infection and induced neural cell death via a drug repurposing screen. Nature medicine 22, 1101-1107.
Zhang, L.X., Zhao, H.J., Sun, D.L., Gao, S.L., Zhang, H.M., and Ding, X.G. (2017). Niclosamide attenuates inflammatory cytokines via the autophagy pathway leading to improved outcomes in renal ischemia/reperfusion injury. Molecular medicine reports 16, 1810-1816.
Zhu, P.J., Hobson, J.P., Southall, N., Qiu, C., Thomas, C.J., Lu, J., Inglese, J., Zheng, W., Leppla, S.H., Bugge, T.H., et al. (2009). Quantitative high-throughput screening identifies inhibitors of anthrax-induced cell death. Bioorganic & medicinal chemistry 17, 5139-5145.

## Claims

1. A composition, comprising particles comprising at least 90% niclosamide by weight, or a pharmaceutically acceptable salt, hydrate, or solvate thereof, wherein the particles have:
(i) a mean bulk density (not tapped) of less than 0.40 g/cm³; and/or
(ii) a mean tapped density of less than 0.55 g/cm³.

2. The composition of claim 1, wherein the particles have a mean bulk density (not tapped) of less than 0.40 g/cm³.

3. The composition of claim 1 or 2, wherein the particles have a mean tapped density of less than 0.55 g/cm³.

4. The composition of any one of claims 1-3, wherein the particles have (a) a mean bulk density (not tapped) of between about 0.02 g/cm³ and 0.30 g/cm³ and a mean tapped density of between about 0.02 g/cm³ and 0.30 g/cm³.

5. The composition of any one of claims 1-4, wherein the particles have a specific surface area (SSA) of at least 3.6 m²/g.

6. The composition of any one of claims 1-5, wherein the particles have a specific surface area (SSA) of between 3.6 m²/g and about 50 m²/g.

7. The composition of any one of claims 1-6, wherein the particles have:
(i) a mean bulk density (not tapped) of less than 0.40 g/cm³;
(ii) a mean tapped density of less than 0.55 g/cm³; and
(iii) a specific surface area (SSA) of at least 3.6 m²/g.

8. The composition of any one of claims 1-7, wherein the particles have a mean particle size by volume distribution of between about of between about 0.1 µm and about 10.5 µm, of between about 0.1 µm and about 8 µm, between about 0.3 µm and about 7µm, between about 0.5 µm and about 6 µm.

9. The composition of any one of claims 1-8, wherein the particles comprise at least 95% niclosamide by weight.

10. The composition of any one of claims 1-9, wherein the composition comprises a suspension further comprising a pharmaceutically acceptable aqueous carrier.

11. The composition of any one of claims 1-10, formulated for pulmonary, intramuscular, subcutaneous, or intraperitoneal administration.

12. The composition of any one of claims 1-11, wherein the niclosamide particles or suspensions thereof are formulated as an dry powder inhaler aerosol, a nebulized suspension, or wherein the niclosamide particles or suspensions thereof are suspended in a suitable propellant system (including but not limited to hydrofluoroalkanes (HFAs)) containing at least one liquefied gas in a pressurized container sealed with a metering valve.

13. The composition of any one of claims 1-12 for use in the treatment of or for use in limiting development of a disorder selected from the group consisting of parasitic infections, viral infections (including but not limited to including to coronaviruses (SARS, MERS, and SARS-CoV-2)), influenza, Ebola virus, Lassa virus, Zika virus, Dengue virus, West Nile and Japanese encephalitis virus, Chikungunya virus, Sindbis virus, Ross River virus, Semliki forest virus, Yellow Fever virus, Rabies virus, herpes simplex virus, hepatitis C virus, rhinovirus, and coxsackivirus, bacterial infections (including but not limited to drug-resistant Mycobacterium tuberculosis, Mycobacterium abscessus, methicillin-resistant Staphylococcus aureus and biofilms, Pseudomonas aeruginosa and biofilms, vancomycin-resistant enterococci, multidrug resistant gram-negative bacteria, and anthrax), asthma, chronic obstructive pulmonary disease (COPD), cystic fibrosis, bronchiectasis, pulmonary and renal fibrosis, pulmonary hypertension, idiopathic pulmonary arterial hypertension (IPAH), acute lung injury (ALI), acute respiratory distress syndrome (ARDS), eosinophilic esophagitis, stroke, ischemic/reperfusion injury, pain, , psoriasis and atopic dermatitis, rheumatoid arthritis, graft-versus host disease and systemic sclerosis, secretory diarrhea, diabetes, kidney disease (including ADPKD), endometriosis, and tocolytic for spontaneous preterm labor;
preferably wherein the subject is a human subject;
preferably wherein the composition is administered via the pulmonary, intramuscular, or subcutaneous route, preferably wherein the composition is administered via the pulmonary route,
preferably wherein the pulmonary administration comprises (a) dry powder inhalation using a suitable dry powder inhaler device, or (b) wherein the pulmonary administration comprises nebulization, and wherein the nebulization results in pulmonary delivery to the subject of aerosol droplets of the particles or suspension thereof.

14. A method for making compound particles, comprising:
(a) introducing (i) a solution comprising at least one solvent selected from the group consisting of acetone, ethanol, methanol, dichloromethane, hexafluroisoproryol alcohol, trifluroethanol, dimethylsulfoxide, tetrahydrofuran (THF), dimethylformamide or combinations thereof, and at least one solute comprising niclosamide or a pharmaceutically acceptable salt, hydrate, or solvate thereof into a nozzle inlet; and (ii) a compressed fluid into an inlet of a vessel defining a pressurizable chamber wherein the compressed fluid is super critical carbon dioxide;
(b) passing the solution out of a nozzle orifice and into the pressurizable chamber to produce an output stream of atomized droplets, wherein the nozzle orifice is located between 2 mm and 20 mm from a sonic energy source located within the output stream, wherein the sonic energy source produces sonic energy with an amplitude between 10% and 100% during the passing, and wherein the nozzle orifice has a diameter of between 20 µm and 125 µm;
(c) contacting the atomized droplets with the compressed fluid, to cause depletion of the solvent from the atomized droplets, to produce compound particles as recited in any one of claims 1-9,
wherein steps (a), (b), and (c) are carried out under supercritical temperature and pressure for the compressed fluid.

15. The method according to claim 14, further comprising:
(d) contacting the compound particles produced in step (c) with an anti-solvent to cause further depletion of the solvent from the compound particles, wherein the anti-solvent is super critical carbon dioxide, wherein step (d) is carried out under supercritical temperature and pressure for the anti-solvent.

## Patentansprüche

1. Zusammensetzung, die Partikel aufweist, die mindestens 90 Gew.-% Niclosamid oder ein pharmazeutisch zulässiges Salz, Hydrat oder Solvat davon aufweist, wobei die Partikel aufweisen:
(i) eine mittlere Schüttdichte (nicht gerüttelt) von weniger als 0,40 g/cm³; und/oder
(ii) eine mittlere gerüttelte Schüttdichte von weniger als 0,55 g/cm³.

2. Zusammensetzung nach Anspruch 1, wobei die Partikel eine mittlere Schüttdichte (nicht gerüttelt) von weniger als 0,40 g/cm³ aufweisen.

3. Zusammensetzung nach Anspruch 1 oder 2, wobei die Partikel eine mittlere gerüttelte Dichte von weniger als 0,55 g/cm³ aufweisen.

4. Zusammensetzung nach einem der Ansprüche 1 bis 3, wobei die Partikel (a) eine mittlere Schüttdichte (nicht gerüttelt) zwischen etwa 0,02 g/cm³ und 0,30 g/cm³ und eine mittlere gerüttelte Schüttdichte zwischen etwa 0,02 g/cm³ und 0,30 g/cm³ aufweisen.

5. Zusammensetzung nach einem der Ansprüche 1 bis 4, wobei die Partikel eine spezifische Oberfläche (SSA) von mindestens 3,6 m²/g aufweisen.

6. Zusammensetzung nach einem der Ansprüche 1 bis 5, wobei die Partikel eine spezifische Oberfläche (SSA) zwischen 3,6 m²/g und etwa 50 m²/g aufweisen.

7. Zusammensetzung nach einem der Ansprüche 1 bis 6, wobei die Partikel aufweisen:
(i) eine mittlere Schüttdichte (nicht gerüttelt) von weniger als 0,40 g/cm³;
(ii) eine mittlere gerüttelte Schüttdichte von weniger als 0,55 g/cm³; und
(iii) eine spezifische Oberfläche (SSA) von mindestens 3,6 m²/g.

8. Zusammensetzung nach einem der Ansprüche 1 bis 7, wobei die Partikel eine volumenbezogene mittlere Partikelgrößenverteilung zwischen etwa 0,1 µm und etwa 10,5 µm, zwischen etwa 0,1 µm und etwa 8 µm, zwischen etwa 0,3 µm und etwa 7 µm sowie zwischen etwa 0,5 µm und etwa 6 µm aufweisen.

9. Zusammensetzung nach einem der Ansprüche 1 bis 8, wobei die Partikel mindestens 95 Gew.-% Niclosamid aufweisen.

10. Zusammensetzung nach einem der Ansprüche 1 bis 9, wobei die Zusammensetzung eine Suspension aufweist, die ferner einen pharmazeutisch zulässigen wässrigen Träger aufweist.

11. Zusammensetzung nach einem der Ansprüche 1 bis 10, formuliert zur pulmonalen, intramuskulären, subkutanen oder intraperitonealen Verabreichung.

12. Zusammensetzung nach einem der Ansprüche 1 bis 11, wobei die Niclosamid-Partikel oder deren Suspensionen als Trockenpulver-Inhalationsaerosol oder als vernebelte Suspension formuliert sind, oder wobei die Niclosamid-Partikel oder deren Suspensionen in einem geeigneten Treibmittelsystem (einschließlich, jedoch nicht beschränkt auf Hydrofluoralkane (HFAs)), das mindestens ein verflüssigtes Gas enthält, in einem unter Druck stehenden, mit einem Dosierventil verschlossenen Behälter suspendiert sind.

13. Zusammensetzung nach einem der Ansprüche 1 bis 12 zur Verwendung bei der Behandlung oder zur Begrenzung der Entwicklung einer Erkrankung, ausgewählt aus der Gruppe bestehend aus parasitären Infektionen, Virusinfektionen (die Coronaviren (SARS, MERS und SARS-CoV-2) aufweisen), Influenza, Ebola-Virus, Lassa-Virus, Zika-Virus, Dengue-Virus, West-Nil-Virus und Japanisches Enzephalitis-Virus, Chikungunya-Virus, Sindbis-Virus, Ross-River-Virus, Semliki-Forest-Virus, Gelbfieber-Virus, Tollwut-Virus, Herpes-simplex-Virus, Hepatitis-C-Virus, Rhinovirus und Coxsackievirus, bakteriellen Infektionen (einschließlich, jedoch nicht beschränkt ist auf arzneimittelresistente Mycobacterium tuberculosis, Mycobacterium abscessus, Methicillin-resistente Staphylococcus aureus und Biofilme, Pseudomonas aeruginosa und Biofilme, Vancomycinresistente Enterokokken, multiresistente gramnegative Bakterien und Anthrax), Asthma, chronisch obstruktive Lungenerkrankung (COPD), Mukoviszidose, Bronchiektasie, Lungen- und Nierenfibrose, pulmonale Hypertonie, idiopathische pulmonale arterielle Hypertonie (IPAH), akutes Lungenversagen (ALI), akutes Atemnotsyndrom (ARDS), eosinophile Ösophagitis, Schlaganfall, ischämische/Reperfusionsschädigung, Schmerzen, Psoriasis und atopische Dermatitis, rheumatoide Arthritis, Graft-versus-Host-Reaktion und systemische Sklerose, sekretorische Diarrhoe, Diabetes, Nierenerkrankungen (die ADPKD aufweisen), Endometriose sowie als Tokolytikum bei spontanen vorzeitigen Wehen;
wobei es sich vorzugsweise bei dem Probanden um einen menschlichen Probanden handelt;
wobei die Zusammensetzung vorzugsweise auf pulmonalem, intramuskulärem oder subkutanem Weg verabreicht wird, wobei die Zusammensetzung auf vorzugsweise pulmonalem Weg verabreicht wird, wobei die pulmonale Verabreichung vorzugsweise (a) eine Trockenpulverinhalation unter Verwendung einer geeigneten Trockenpulverinhalationsvorrichtung aufweist, oder (b) wobei die pulmonale Verabreichung eine Vernebelung aufweist, und wobei die Vernebelung zu einer pulmonalen Abgabe von Aerosoltröpfchen der Partikel oder einer Suspension davon an den Probanden führt.

14. Verfahren zum Herstellen von Verbundpartikeln, das aufweist:
(a) Einleiten (i) einer Lösung, die mindestens ein Lösungsmittel, das aus der Gruppe ausgewählt ist, die aus Aceton, Ethanol, Methanol, Dichlormethan, Hexafluorisopropanol, Trifluorethanol, Dimethylsulfoxid, Tetrahydrofuran (THF), Dimethylformamid oder Kombinationen davon besteht, und mindestens einen gelösten Stoff aufweist, der Niclosamid oder ein pharmazeutisch zulässiges Salz, Hydrat oder Solvat davon aufweist, in einen Düseneinlass; und (ii) eines komprimierten Fluids in einen Einlass eines Behälters, der eine unter Druck setzbare Kammer definiert, wobei das komprimierte Fluid überkritisches Kohlendioxid ist;
(b) Leiten der Lösung aus einer Düsenöffnung in die unter Druck setzbare Kammer, um einen Ausgangsstrom aus zerstäubten Tröpfchen zu erzeugen, wobei sich die Düsenöffnung in einem Abstand zwischen 2 mm und 20 mm von einer Schallenergiequelle befindet, die innerhalb des Ausgangsstroms angeordnet ist, wobei die Schallenergiequelle während des Durchflusses Schallenergie mit einer Amplitude zwischen 10 % und 100 % erzeugt, und wobei die Düsenöffnung einen Durchmesser zwischen 20 µm und 125 µm aufweist;
(c) Inkontaktbringen der zerstäubten Tröpfchen mit dem komprimierten Fluid, um eine Entfernung des Lösungsmittels aus den zerstäubten Tröpfchen zu bewirken, um Verbundpartikel nach einem der Ansprüche 1 bis 9 herzustellen, wobei die Schritte (a), (b) und (c) bei überkritischer Temperatur und überkritischem Druck für das komprimierte Fluid durchgeführt werden.

15. Verfahren nach Anspruch 14, das aufweist:
(d) Das Inkontaktbringen der in Schritt (c) hergestellten Verbundpartikel mit einem Antisolvent, um eine weitere Entfernung des Lösungsmittels aus den Verbundpartikeln zu bewirken, wobei das Antisolvent überkritisches Kohlendioxid ist, wobei Schritt (d) bei überkritischer Temperatur und überkritischem Druck für das Antisolvent durchgeführt wird.

## Revendications

1. Composition comprenant des particules contenant au moins 90 % en poids de niclosamide, ou un sel, un hydrate ou un solvate pharmaceutiquement acceptable de celui-ci, où les particules ont :
(i) une densité apparente moyenne (non tassée) inférieure à 0,40 g/cm³ ; et/ou
(ii) une densité apparente moyenne (après tassement) inférieure à 0,55 g/cm³.

2. Composition selon la revendication 1, où les particules ont une densité apparente moyenne (non tassée) inférieure à 0,40 g/cm³.

3. Composition selon la revendication 1 ou la revendication 2, où les particules ont une densité apparente moyenne (après tassement) inférieure à 0,55 g/cm³.

4. Composition selon l'une des revendications 1 à 3, où les particules ont (a) une densité apparente moyenne (non tassée) comprise entre 0,02 g/cm³ environ et 0,30 g/cm³ et une densité apparente moyenne (après tassement) comprise entre 0,02 g/cm³ environ et 0,30 g/cm³.

5. Composition selon l'une des revendications 1 à 4, où les particules ont une surface spécifique (SSA) d'au moins 3,6 m²/g.

6. Composition selon l'une des revendications 1 à 5, où les particules ont une surface spécifique (SSA) comprise entre 3,6 m²/g et 50 m²/g environ.

7. Composition selon l'une des revendications 1 à 6, où les particules présentent :
(i) une densité apparente moyenne (non tassée) inférieure à 0,40 g/cm³ ;
(ii) une densité apparente moyenne après tassement inférieure à 0,55 g/cm³; et
(iii) une surface spécifique (SSA) d'au moins 3,6 m²/g.

8. Composition selon l'une des revendications 1 à 7, où les particules présentent une taille moyenne par distribution en volume comprise entre 0,1 µm environ et 10,5 µm environ, entre 0,1 µm environ et 8 µm environ, entre 0,3 µm environ et 7 µm environ, entre 0,5 µm environ et 6 µm environ.

9. Composition selon l'une des revendications 1 à 8, où les particules comprennent au moins 95 % en poids de niclosamide.

10. Composition selon l'une des revendications 1 à 9, où la composition comprend une suspension comprenant en outre un véhicule aqueux pharmaceutiquement acceptable.

11. Composition selon l'une des revendications 1 à 10, formulée pour une administration pulmonaire, intramusculaire, sous-cutanée ou intrapéritonéale.

12. Composition selon l'une des revendications 1 à 11, où les particules de niclosamide ou des suspensions de celles-ci sont formulées comme aérosol pour inhalateur à poudre sèche, suspension nébulisée, ou où les particules de niclosamide ou des suspensions de celles-ci sont mises en suspension dans un système propulseur approprié (comprenant, sans limitation à ceux-ci, des hydrofluoroalcanes (HFA)) contenant au moins un gaz liquéfié dans un récipient sous pression obturé par une vanne de dosage.

13. Composition selon l'une des revendications 1 à 12, destinée à être utilisée dans le traitement, ou pour limiter le développement d'une pathologie sélectionnée dans le groupe constitué par des infections parasitaires, des infections virales (y compris, sans limitation à celles-ci, des coronavirus (SARS, MERS et SARS-CoV-2)), la grippe, le virus Ebola, le virus Lassa, le virus Zika, le virus de la dengue, le virus du Nil occidental et le virus de l'encéphalite japonaise, le virus Chikungunya, le virus de Sindbis, le virus de Ross River, le virus de la forêt de Semliki, le virus de la fièvre jaune, le virus de la rage, le virus de l'herpès simplex, le virus de l'hépatite C, le rhinovirus et le virus Coxsackie, les infections bactériennes (y compris, sans limitation à celles-ci, Mycobacterium tuberculosis résistant aux médicaments, Mycobacterium abscessus, Staphylococcus aureus et des biofilms résistants à la méticilline, Pseudomonas aeruginosa et des biofilms, entérocoques résistants à la vancomycine, des bactéries Gram négatives multirésistantes et l'anthrax), l'asthme, la bronchopneumopathie chronique obstructive (BPCO), la mucoviscidose, les bronchectasies, la fibrose pulmonaire et rénale, l'hypertension pulmonaire, l'hypertension artérielle pulmonaire idiopathique (HAPI), la lésion pulmonaire aiguë (ALI), le syndrome de détresse respiratoire aiguë (SDRA), l'œsophagite éosinophile, les accidents vasculaires cérébraux, l'ischémie/reperfusion, les douleurs, le psoriasis et la dermatite atopique, la polyarthrite rhumatoïde, la réaction du greffon contre l'hôte et la sclérose systémique, la diarrhée sécrétoire, le diabète, l'insuffisance rénale (y compris la polykystose rénale auto-induite), l'endométriose, et comme tocolytique pour la menace d'accouchement prématuré ;
où de préférence le sujet est un sujet humain ;
où de préférence la composition est administrée par voie pulmonaire, intramusculaire ou sous-cutanée, où de préférence la composition est administrée par voie pulmonaire,
où de préférence l'administration pulmonaire comprend (a) l'inhalation de poudre sèche au moyen d'un dispositif d'inhalation de poudre sèche approprié, ou (b) où l'administration pulmonaire comprend une nébulisation, et où la nébulisation entraîne l'administration pulmonaire au sujet de gouttelettes d'aérosol des particules ou d'une suspension de celles-ci.

14. Procédé de fabrication de particules composites, comprenant :
(a) l'introduction (i) d'une solution comprenant au moins un solvant sélectionné dans le groupe constitué par l'acétone, l'éthanol, le méthanol, le dichlorométhane, l'alcool hexafluoro-isopropylique, le trifluoroéthanol, le diméthylsulfoxyde, le tétrahydrofurane (THF), le diméthylformamide ou des combinaisons de ceux-ci, et au moins un soluté comprenant du niclosamide ou un sel, un hydrate ou un solvate pharmaceutiquement acceptable de celui-ci dans un orifice d'admission ; et (ii) un fluide comprimé dans une entrée d'un récipient définissant une chambre pressurisable, où le fluide comprimé est du dioxyde de carbone supercritique ;
(b) la sortie de la solution par un orifice d'embout vers la chambre pressurisable pour produire un flux de sortie de gouttelettes atomisées, ledit orifice d'embout étant situé entre 2 mm et 20 mm d'une source d'énergie sonique disposée à l'intérieur du flux de sortie, ladite source d'énergie sonique produisant une énergie sonique d'une amplitude comprise entre 10 % et 100 % pendant la sortie, et l'orifice d'embout ayant un diamètre compris entre 20 µm et 125 µm ;
(c) la mise en contact des gouttelettes atomisées avec le fluide comprimé, afin de provoquer l'élimination du solvant des gouttelettes atomisées, pour produire des particules de composé selon l'une des revendications 1 à 9, où les étapes (a), (b) et (c) sont exécutées à une température et sous une pression supercritiques pour le fluide comprimé.

15. Procédé selon la revendication 14, comprenant en outre :
(d) la mise en contact des particules de composé produites lors de l'étape (c) avec un antisolvant afin de provoquer une élimination supplémentaire du solvant des particules de composé, ledit antisolvant étant du dioxyde de carbone supercritique, et l'étape (d) étant exécutée à une température et sous une pression supercritiques pour l'antisolvant.
